(19) Europäisches Patentamt
European Patent Office
Office européen des brevets

(11) EP 2 913 404 B1

(12) EUROPEAN PATENT SPECIFICATION

(45) Date of publication and mention
of the grant of the patent:
04.04.2018 Bulletin 2018/14

(51) Int Cl.:
*C12Q 1/04* (2006.01)

(21) Application number: 15161700.8

(22) Date of filing: 19.02.2008

(54) SYSTEMS AND METHODS FOR PRESUMPTIVE IDENTIFICATION OF MICROORGANISM TYPE IN A CULTURE

SYSTEME UND VERFAHREN ZUR PRÄSUMPTIVEN IDENTIFIZIERUNG EINES MIKROORGANISMUSTYPS IN EINER KULTUR

SYSTÈMES ET PROCÉDÉS D'IDENTIFICATION PRÉSUMÉE DE TYPE DE MICRO-ORGANISMES DANS UNE CULTURE

(84) Designated Contracting States:
AT BE BG CH CY CZ DE DK EE ES FI FR GB GR HR HU IE IS IT LI LT LU LV MC MT NL NO PL PT RO SE SI SK TR

(43) Date of publication of application:
02.09.2015 Bulletin 2015/36

(62) Document number(s) of the earlier application(s) in accordance with Art. 76 EPC:
08779567.0 / 2 250 281

(73) Proprietor: Becton, Dickinson and Company
Franklin Lakes, NJ 07417-1880 (US)

(72) Inventor: Beaty, Patrick Shawn
Dallastown, PA 17313 (US)

(74) Representative: dompatent von Kreisler Selting Werner -
Partnerschaft von Patent- und Rechtsanwälten mbB
Deichmannhaus am Dom
Bahnhofsvorplatz 1
50667 Köln (DE)

(56) References cited:
EP-A2- 0 697 460         WO-A2-2005/103284
US-A- 5 814 474          US-A1- 2002 155 424
US-A1- 2007 032 706

**Description**

**1 FIELD OF THE INVENTION**

Disclosed are improved systems and methods for identifying a microorganism type in a culture.

**2 BACKGROUND OF THE INVENTION**

Rapid and reliable detection of microorganisms in a culture, such as a blood culture, is among the most important functions of the clinical microbiology laboratory. Currently, the presence of biologically active agents such as bacteria in a patient's body fluid, and especially in blood, is determined using culture vials. A small quantity of the patient's body fluid is injected through an enclosing rubber septum into a sterile vial containing a culture medium and the vial is then incubated and monitored for microorganism growth.

Common visual inspection of the culture vial then involves monitoring the turbidity or observing eventual color changes of the liquid suspension within the vial. Known instrument methods can also be used to detect changes in the carbon dioxide content of the culture vessels, which is a metabolic byproduct of the bacterial growth. Monitoring the carbon dioxide content can be accomplished by methods well established in the art.

In some instances, non-invasive infrared microorganism detection instrument is used in which special vials having infrared-transmitting windows are utilized. In some instances, glass vials are transferred to an infrared spectrometer by an automated manipulator arm and measured through the glass vial. In some instances, chemical sensors are disposed inside the vial. These sensors respond to changes in the carbon dioxide concentration in the liquid phase by changing their color or by changing their fluorescence intensity. These techniques are based on light intensity measurements and require spectral filtering in the excitation and/or emission signals.

As the above indicates, several different culture systems and approaches are available to laboratories. For example, the BACTEC® radiometric and nonradiometric systems (Becton Dickenson Diagnostic Instrument Systems, Sparks, Maryland) are often used for this task. The BACTEC® 9240 instrument, for example, accommodates up to 240 culture vessels and serves as an incubator, agitator, and detection system. Each vessel contains a fluorescent $CO_2$ sensor, and the sensors are monitored on a continuous basis (e.g., every ten minutes). Cultures are recognized as positive by computer algorithms for growth detection based on an increasing rate of change as well as sustained increase in $CO_2$ production rather than by the use of growth index threshold or delta values. The BACTEC® 9240 is completely automated once the vessels have been loaded.

A drawback with these microorganism detection approaches is that they do not always detect microorganism type in such cultures. What is needed in each of the above-identified systems is a way to non-invasively and automatically determine what type of microorganism is in a culture. U.S. Patent Publication No. 2002/0155424 to Pitner et al. describes an identifying/typing method for microorganisms that employs a normalization procedure. In Pitneret et al. the fluorescence serves as an oxygen detector, with a decrease in fluorescence associated with an increase in oxygen.

European Patent No. 0697460 describes the use of an intensity modulated light source to illuminate the chemical sensor materials. The fluorescence component is split into its high frequency and DC components. The ratio of those components is used to measure the response of the sensor materials to different chemical parameters.

**3 SUMMARY OF THE INVENTION**

To meet the needs identified in the prior art, methods, and apparatus for presumptive organism identification are provided for any culture system designed to culture a sample for the presence of microorganisms of an unknown type with an intended use of determining either the presence or the presence and identification of the micrganism type. The invention is as disclosed in the appended claims. Advantageously, using the novel methods, and apparatus of the present invention, an incubating system, such as the BACTEC® blood culture system, can be programmed to determine the microorganism type in a culture before manual tests, such as a Gram stain or a subculture, are performed. In fact, in some instances, the novel methods, and apparatus of the present invention can obviate any need to perform a Gram test or a subculture in order to identify the microorganism type that is infecting a culture.

Briefly, novel parameters disclosed herein, such as a maximum metabolic rate and an extent of growth, are used to determine the type of microorganisms that is infecting a culture. It has been unexpectedly discovered that each microorganism type (*e.g.*, each microorganism species) adopts unique values for these novel parameters. Based on this unexpected discovery, the microorganism type infecting a biological sample can be determined directly by an automated incubator before manual tests, such as a Gram stain or a subculture, are performed by a lab technician. In fact, in some instances such manual tests are no longer required to determine the microorganism type infecting a culture.

In the present invention, values for the novel parameters disclosed herein from each biological sample that has been infected with a microorganism of known type are computed and used to populate an optional lookup table or to train a classifier or other form of equation that can be used to determine the identity of microorganism types. Typically, the lookup table is built using microorganisms of known species. The values of each of the microorganism types (*e.g.*, microorganism species) that could potentially infect an unknown biological sample are computed and used to populate the lookup table. Advantageously, because the novel parameters, such as a maximum metabolic rate and an extent of growth, are computed using observables that are already recorded in conventional laboratories that work with biological samples, such a lookup table can be assembled without any requirement for running additional experiments. For example, in many laboratories, over time, several infected samples are identified using conventional Gram stains and/or subcultures. Moreover, in such laboratories, the growth curve data that tracks metabolism as a function of time has been recorded for such samples. Indeed, it is often such growth curve data that leads to the determination that such samples were infected with microorganisms. For these infected samples, the novel parameters can be computed from the growth curve data, matched with the microorganism type from the Gram stains and/or subcultures, and used to populate a lookup table. Then, the type of microorganism type infecting a new culture (an unknown) can be determined by computing the values of the novel parameters for the new culture and, for example, comparing them to the values in the lookup table for a given microorganism type.

While numerous exemplary values for the novel parameters disclosed herein are given in the data presented herein for many different microorganism types for a given media, it is to be appreciated that these values for the novel parameters for any given microorganism type may change when the media used to support growth of the culture is altered. Therefore, for any given lookup table, care should be taken to make sure that the biological media used to determine the growth curve of the unknown culture is the same or similar to the biological media used to determined the growth curve of the known samples used to populate the lookup table. Moreover, it is possible that the values of the novel parameters may vary when a different incubator is used. Thus, preferentially, the same incubator used to generate data used to populate the lookup table is also used for the unknown culture. Alternatively, several different lookup tables, where each lookup table is for a different media type and/or incubator, can be constructed.

There is disclosed herein exemplary values for the novel parameters, such as maximum metabolic rate and extent of growth, from several different microorganism types for a given growth medium. The data show that each microorganism type has a different unique and characteristic value for the novel parameters disclosed herein. Moreover, the values for the novel parameters were computed from growth data that was previously recorded for the samples and thus no additional experimentation was required to compute the values presented herein. As discussed above, the values disclosed herein for the novel parameters may shift when different media is used. However, as the data disclosed herein clearly demonstrates, for a given media, the values for the novel parameters will be different for each microorganism type and thus can be used as a basis for identifying the microorganism type in an unknown sample that has been presumptively identified as being infected with a microorganism type. As such, the systems and methods of the present invention can provide a number of applications useful in microbiology and related fields, and finds particular application in cell culture sterility test procedures.

In one aspect, the present invention utilizes the differences in rate of growth and extent of growth to provide information about the microorganism type present and growing in a culture that can result in or contribute to the identification of the microorganism type. This aspect of the present invention utilizes a data transformation that can be applied to metabolic or cell growth data in a way that provides values for parameters for the microorganism type present in the culture. This aspect of the present invention can be used to determine the identity of the microorganism type.

In another aspect, the present invention provides a method of identifying a microorganism type in a culture in a vessel. In the method, a normalization relative value is calculated for each respective measurement in a plurality of measurements of a biological state of the culture in the vessel between (i) the respective measurement and (ii) an initial biological state of the culture taken at an initial time point, thereby forming a plurality of normalization relative values.

The plurality of normalization relative values can be broken down, on a timewise basis, into predetermined fixed intervals of time points between the first time point and the second time point. For instance, a first predetermined fixed interval may include the first ten normalization relative values, a second predetermined fixed interval may include the next ten normalization relative values, and so forth until the second time point is reached. For each of these respective predetermined fixed intervals of time points between the first time point and the second time point, a first derivative of the normalization relative values in the respective predetermined fixed interval is determined, thereby forming a plurality of rate transformation values.

There is a rate transformation value for each predetermined fixed interval of time points. The plurality of rate transformation values can be considered as comprising a plurality of sets of rate transformation values. Each respective set of rate transformation values is for a different set of contiguous time points between the first time point and the second time point. For example, the first set of rate transformation values may be the first seven rate transformation values in the plurality of rate transformation values, the second set of rate transformation values may be the next seven rate transformation values in the plurality of rate transformation values, and so forth. For each respective set of rate transformation values in the plurality of sets of rate transformation values, an average relative transformation value is computed as a measure of central tendency of each of the rate transformation values in the respective set of rate transformation values. In this way, a plurality of average relative transformation values is computed.

A maximum metabolic rate and an extent of growth is determined from the plurality of normalization relative values and the plurality of average relative transformation values. In some embodiments, the maximum metabolic rate and the extent of growth is compared with an optional lookup table that matches the maximum metabolic rate and the extent of growth to a microorganism type, thereby determining the microorganism type in the culture in the vessel. In some embodiments, the maximum metabolic rate and the extent of growth is used in an equation or other form of trained classifier to determine the microorganism type in the culture in the vessel.

In some embodiments, of this disclosure, an identification of the microorganism type in the culture in the vessel is outputted to a user interface device, a monitor, a computer-readable storage medium, a computer-readable memory, or a local or remote computer system. In some embodiments an identification of the microorganism type in the culture is displayed.

In some embodiments, the first time point is five or more minutes after the initial time point and the second time point is thirty or more hours after the initial time point. In some embodiments, the first time point is between 0.5 hours and 3 hours after the initial time point and the second time point is between 4.5 hours and twenty hours after the initial time point. In some embodiments, the measure of central tendency of the rate transformation values in a first set of rate transformation values in the plurality of sets of rate transformation values comprises a geometric mean, an arithmetic mean, a median, or a mode of each of the rate transformation values in the first set of rate transformation values.

In some embodiments, the measurements in the plurality of measurements of the biological state of the culture are each taken of the culture at a periodic time interval between the first time point and the second time point. In some embodiments, the periodic time interval is an amount of time between one minute and twenty minutes, between five minutes and fifteen minutes, or between 0.5 minutes and 120 minutes.

In some embodiments, the initial biological state of the culture is determined by a fluorescence output of a sensor that is in contact with the culture. For instance, in some embodiments, the amount of fluorescence output of the sensor is affected by $CO_2$ concentration, $O_2$ concentration, or pH.

In some embodiments, between 10 and 50,000 measurements, between 100 and 10,000 measurements, or between 150 and 5,000 measurements of the biological state of the culture in the vessel are in the plurality of measurements of the biological state of the culture. In some embodiments, each respective predetermined fixed interval of time points comprises or consists of each of the rate transformation values for time points in a time window between the first time point and the second time point, where the time window is a period of time that is between twenty minutes and ten hours, twenty minutes and two hours, or thirty minutes and ninety minutes.

In some embodiments, each set of rate transformation values in the plurality of rate transformation values comprises or consists of between four and twenty, between five and fifteen, or between 2 and 100 contiguous rate transformation values. In some embodiments, there are between five and five hundred or between twenty and one hundred average relative transformation values in the plurality of average relative transformation values. In some embodiments, a volume of the culture is between 1 ml and 40 ml, between 2 ml and 10 ml, less than 100ml, or greater than 100 ml.

In some embodiments, the vessel comprises a sensor composition in fluid communication with the culture, where the sensor composition comprises a luminescent compound that exhibits a change in luminescent property, when irradiated with light containing wavelengths that cause said luminescent compound to luminesce, upon exposure to oxygen, and where the presence of the sensor composition is non-destructive to the culture and where the initial biological state of the culture is measured by the method of (i) irradiating the sensor composition with light containing wavelengths that cause the luminescent compound to luminesce and (ii) observing the luminescent light intensity from the luminescent compound while irradiating the sensor composition with the light. In some embodiments, the luminescent compound is contained within a matrix that is relatively impermeable to water and non-gaseous solutes, but which has a high permeability to oxygen. In some embodiments, the matrix comprises rubber or plastic.

In some embodiments, the maximum metabolic rate is deemed to be a maximum average relative transformation value in the plurality of average relative transformation values. In some embodiments, the extent of growth is determined by the equation:

$$NR_{after\_growth} - NR_{minimum\_growth}$$

where $NR_{after\_growth}$ is a normalization relative value in the plurality of normalization relative values that was used in the calculation of (i) the first average relative transformation value following the maximum average relative transformation value, (ii) the maximum average relative transformation value, or (iii) the first average relative transformation value preceding the maximum average relative transformation value in the plurality of average relative transformation values, and $NR_{minimum\_growth}$ is a normalization relative value in the plurality of normalization relative values that was used in the calculation of the first average relative transformation value to achieve a threshold value. In some embodiments, the threshold value is a value between 5 and 100 or between 25 and 75.

In some embodiments, the extent of growth is determined by the equation:

$$\text{ARTmax} * (\text{time}_{\text{ARTmax}} - \text{time}_{\text{initial}})$$

where ARTmax is a maximum average relative transformation value in the plurality of average relative transformation values, $\text{time}_{\text{ARTmax}}$ is a duration of time between (a) the initial time point and (b) a time point when the normalization relative value in the plurality of normalization relative values that was used in the calculation of (i) the first average relative transformation value following the maximum average relative transformation value, (ii) the maximum average relative transformation value, or (iii) the first average relative transformation value preceding the maximum average relative transformation value in the plurality of average relative transformation values was measured, and $\text{time}_{\text{initial}}$ is a duration of time between (i) the initial time point and (ii) a time point when the normalization relative value in the plurality of normalization relative values that was used in the calculation of the first average relative transformation value to achieve a threshold value was measured. In some embodiments, the threshold value is a value between 5 and 100 or between 25 and 75.

In some embodiments, the extent of growth is determined by the equation:

$$[\text{ARTmax} * (\text{time}_{\text{ARTmax}} - \text{time}_{\text{initial}})] / \text{time}_{\text{initial}}$$

where ARTmax is a maximum average relative transformation value in the plurality of average relative transformation values, $\text{time}_{\text{ARTmax}}$ is a duration of time between (a) the initial time point and (b) a time point when the normalization relative value in the plurality of normalization relative values that was used in the calculation of (i) the first average relative transformation value following the maximum average relative transformation value, (ii) the maximum average relative transformation value, or (iii) the first average relative transformation value preceding the maximum average relative transformation value in the plurality of average relative transformation values was measured, and $\text{time}_{\text{initial}}$ is a duration of time between (i) the initial time point and (ii) a time point when the normalization relative value in the plurality of normalization relative values that was used in the calculation of the first average relative transformation value to achieve a threshold value was measured.

In some embodiments, the determining step identifies the microorganism type in the culture in the vessel as (i) a bacterium in the Enterobacteriaceae family or (ii) a bacterium not in the Enterobacteriaceae family based upon the maximum metabolic rate and the extent of growth. In some embodiments, the determining step identifies the microorganism type as bacteria based upon the maximum metabolic rate and the extent of growth. In some embodiments, the determining step identifies the microorganism type as (i) Enterobacteriaceae, (ii) Staphylococcaceae, (iii) Streptococcus, or (iv) Acinetobacter based upon the maximum metabolic rate and the extent of growth. In some embodiments, the determining step identifies the microorganism type as a single genera of the Enterobacteriaceae selected from the group consisting of *Alishewanella, Alterococcus, Aquamonas, Aranicola, Arsenophonus, Azotivirga, Blochmannia, Brenneria, Buchnera, Budvicia, Buttiauxella, Cedecea, Citrobacter, Dickeya, Edwardsiella, Enterobacter, Erwinia, Escherichia, Ewingella, Griimontella, Hafnia, Klebsiella, Kluyvera, Leclercia, Leminorella, Moellerella, Morganella, Obesumbacterium, Pantoea, Pectobacterium, Candidatus Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia,* and *Yokenella.*

In some embodiments, the determining step identifies the microorganism type as a single species of Staphylococcaceae selected from the group consisting of *Staphylococcus aureus, Staphylococcus caprae, Staphylococcus epidermidis, Staphylococcus haemolyticus, Staphylococcus hominis, Staphylococcus lugdunensis, Staphylococcus pettenkoferi, Staphylococcus saprophyticus, Staphylococcus warneri,* and *Staphylococcus xylosus* bacteria. In some embodiments, the determining step identifies the microorganism type as a single species of Streptococcus selected from the group consisting of *S. agalactiae, S. bovis, S. mutans, S. pneumoniae, S. pyogenes, S. salivarius, S. sanguinis, S. suis, Streptococcus viridans,* and *Streptococcus uberis.*

In some embodiments, the determining step identifies the microorganism type as aerobic based upon the maximum metabolic rate and the extent of growth. In some embodiments, the determining step identifies the microorganism type as anaerobic based upon the maximum metabolic rate and the extent of growth.

In some embodiments, the initial biological state of the culture is measured by a colorimetric means, a fluorometric means, a nephelometric means, or an infrared means. IN some embodiments, each biological state in the plurality of measurements of the biological state is determined by a colorimetric means, a fluorometric means, a nephelometric means, or an infrared means. In some embodiments, the culture is a blood culture from a subject (*e.g.,* a human subject, a mammalian subject, *etc.*).

In another aspect, the present invention provides an apparatus for identifying a microorganism type in a culture in a vessel in which the apparatus comprises a processor and a memory, coupled to the processor, for carrying out any of the methods disclosed herein. In still another aspect, the present invention provides is a computer-readable medium storing a computer program product for identifying a microorganism type in a culture in a vessel, where the computer program product is executable by a computer. The computer program product comprises instructions for carrying out any of the methods disclosed herein.

Yet another aspect of the present invention provides a method of identifying a microorganism type in a culture in a vessel. A plurality of measurements of the biological state of the culture in the vessel is obtained, each measurement in the plurality of measurements taken at a different time point between a first time point and a second time point. For each respective predetermined fixed interval of time points between the first time point and the second time point, a first derivative of the measurements of the biological state in the respective predetermined fixed interval of time points is determined, thereby forming a plurality of rate transformation values, where the plurality of rate transformation values comprises a plurality of sets of rate transformation values, and where each respective set of rate transformation values in the plurality of sets of rate transformation values is for a different set of contiguous time points between the first time point and the second time point. For each respective set of rate transformation values in the plurality of sets of rate transformation values, an average relative transformation value is computed as a measure of central tendency of each of the rate transformation values in the respective set of rate transformation values, thereby computing a plurality of average relative transformation values. A maximum metabolic rate and an extent of growth from the plurality of normalization relative values and the plurality of average relative transformation values is determined. The maximum metabolic rate and the extent of growth is compared with a lookup table that matches the maximum metabolic rate and the extent of growth to a microorganism type, thereby determining the microorganism type in the culture in the vessel.

## 4 BRIEF DESCRIPTION OF THE DRAWINGS

Figure 1 illustrates an apparatus for identifying a microorganism type in a culture in a vessel, the apparatus comprising a processor and a memory, coupled to the processor.

Figure 2 illustrates a schematic drawing of a culture vessel and $CO_2$ detector system.

Figures 3A and 3B illustrate a method for identifying a microorganism type in a culture in a vessel.

Figure 4 shows a plot of normalization relative values measured from a blood culture in a vessel.

Figure 5 is a plot of the average relative transformation values over time based on the average rate of change in rate transformation values of Figure 4 over time.

Figure 6 is the second derivative plot of normalization relative values of Figure 4 and shows the changes in metabolism rate with time.

Figure 7 is a plot of the ratio of the extent of growth / versus maximum metabolic rate for reference cultures containing microorganisms of known type.

Like reference numerals refer to corresponding parts throughout the several views of the drawings.

## 5 DETAILED DESCRIPTION OF THE INVENTION

Methods and apparatus for identifying a microorganism type in a culture are provided. In one aspect, a normalization relative value is calculated for each respective measurement of a biological state of the culture between (i) the respective measurement and (ii) an initial biological state. For each fixed interval of time points, a first derivative of the normalization relative values for measurements of the biological state in the interval of time points is calculated, thereby forming a plurality of rate transformation values. For each respective set of rate transformation values in the plurality of rate transformation values, a measure of central tendency of the rate transformation values in the set is computed, thereby forming a plurality of average relative transformation values. In some embodiments, a maximum metabolic rate and an extent of growth, determined from the normalization relative values and the average relative transformation values, are compared against an optional lookup table that matches these values to a microorganism type.

**5.1 Definitions**

The term "Acinetobacter" as used herein refers to a Gram-negative genus of bacteria belonging to the phylum Proteobacteria. Non-motile, Acinetobacter species are oxidase-negative, and occur in pairs under magnification.

The term "biological state" as used herein refers to a measure of the metabolic activity of a culture as determined by, for example, $CO_2$ concentration, $O_2$ concentration, pH, a rate of change in $CO_2$ concentration, a rate of change in $O_2$ concentration, or a rate of change in pH in the culture.

The term "blood" as used herein means either whole blood or any one, two, three, four, five, six, or seven cell types from the group of cells types consisting of red blood cells, platelets, neutrophils, eosinophils, basophils, lymphocytes, and monocytes. Blood can be from any species including, but not limited to, humans, any laboratory animal (e.g., rat, mouse, dog, chimp), or any mammal.

The term "blood culture" as used herein refers to any amount of blood that has been mixed with blood culture media. Examples of culture media include, but are not limited to, supplemented soybean casein broth, soybean casein digest, hemin, menadione, sodium bicarbonate, sodium polyaneltholesulfonate, sucrose, pyridoxal HCKl, yeast extract, and L-cysteine. One or more reagents that may be used as blood culture media are found, for example, in Stanier et al., 1986, The Microbial World, 5th edition, Prentice-Hall, Englewood Cliffs, New Jersey, pages 10-20, 33-37, and 190-195. In some instances, a blood culture is obtained when a subject has symptoms of a blood infection or bacteremia. Blood is drawn from a subject and put directly into a vessel containing a nutritional broth. In some embodiments, ten milliliters of blood is needed for each vessel.

The term "culture" as used herein refers to any biological sample from a subject that is either in isolation or mixed with one or more reagents that are designed to culture cells. The biological sample from the subject can be, for example, blood, cells, a cellular extract, cerebral spinal fluid, plasma, serum, saliva, sputum, a tissue specimen, a tissue biopsy, urine, a wound secretion, a sample from an in-dwelling line catheter surface, or a stool specimen. The subject can a member of any species including, but not limited to, humans, any laboratory animal (*e.g.*, rat, mouse, dog, chimp), or any mammal. One or more reagents that may be mixed with the biological sample are found, for example, in Stanier et al., 1986, The Microbial World, 5th edition, Prentice-Hall, Englewood Cliffs, New Jersey, pages 10-20, 33-37, and 190-195. A blood culture is an example of a culture. In some embodiments, the biological sample is in liquid form and the amount of the biological sample in the culture is between 1 ml and 150 ml, between 2 ml and 100 ml, between 0.5 ml and 90 ml, between 0.5 ml and 75 ml, or between 0.25 ml and 100,000 ml. In some embodiments, the biological sample is in liquid form and is between 1 and 99 percent of the volume of the culture, between 5 and 80 percent of the volume of the culture, between 10 and 75 percent of the volume of the culture, less than 80 percent of the volume of the culture, or greater than 10 percent of the volume of the culture. In some embodiments, the biological sample is between 1 and 99 percent of the total weight of the culture, between 5 and 80 percent of the total weight of the culture, between 10 and 75 percent of the total weight of the culture, less than 80 percent of the total weight of the culture, or greater than 10 percent of the total weight of the culture.

As used herein, the term "Enterobacteriaceae" refers to a large family of bacteria, including *Salmonella* and *Escherichia coli.* Enterobacteriaceae are also referred to herein as the Enteric group. Genetic studies place them among the Proteobacteria, and they are given their own order (Enterobacteriales). Members of the Enterobacteriaceae are rod-shaped, and are typically 1 $\mu$m to 5 $\mu$m in length. Like other proteobacteria they have Gram-negative stains, and they are facultative anaerobes, fermenting sugars to produce lactic acid and various other end products. They also reduce nitrate to nitrite. Unlike most similar bacteria, Enterobacteriaceae generally lack cytochrome C oxidase, although there are exceptions (e.g. *Plesiomonas*). Most have many flagella, but a few genera are non-motile. They are non-spore forming, and except for *Shigella dysenteriae* strains they are catalase-positive. Many members of this family are a normal part of the gut flora found in the intestines of humans and other animals, while others are found in water or soil, or are parasites on a variety of different animals and plants. Most members of Enterobacteriaceae have peritrichous Type I fimbriae involved in the adhesion of the bacterial cells to their hosts. Genera of the Enterobacteriaceae include, but are not limited to, *Alishewanella*, *Alterococcus*, *Aquamonas*, *Aranicola*, *Arsenophonus*, *Azotivirga*, *Blochmannia*, *Brenneria*, *Buchnera*, *Budvicia*, *Buttiauxella*, *Cedecea*, *Citrobacter*, *Dickeya*, *Edwardsiella*, *Enterobacter*, *Erwinia* (*e.g. Erwinia amylovora*), *Escherichia* (*e.g. Escherichia coli*), *Ewingella*, *Griimontella*, *Hafnia*, *Klebsiella* (*e.g. Klebsiella pneumoniae*), *Kluyvera*, *Leclercia*, *Leminorella*, *Moellerella*, *Morganella*, *Obesumbacterium*, *Pantoea, Pectobacterium*, *Candidatus Phlomobacter*, *Photorhabdus* (*e.g., Photorhabdus luminescens*), *Plesiomonas* (*e.g. Plesiomonas shigelloides*), *Pragia*, *Proteus* (*e.g. Proteus vulgaris*), *Providencia*, *Rahnella, Raoultella, Salmonella, Samsonia, Serratia* (*e.g. Serratia marcescens*), *Shigella, Sodalis, Tatumella, Trabulsiella, Wigglesworthia*, *Xenorhabdus, Yersinia* (*e.g., Yersinia pestis*), and *Yokenella.* More information about Enterobacteriaceae is found in Stanier et al., 1986, The Microbial Would, 5th edition, Prentice-Hall, Englewood Cliffs, New Jersey, Chapter 5.

As used herein, the term "instance" refers to the execution of a step in an algorithm. Some steps in an algorithm may be run several times, with each repeat of the step being referred to as an instance of the step.

As used herein, the term "microorganism" refers to organisms with a diameter of 1 mm or less excluding viruses.

As used herein, the term "microorganism type" refers to any subclassification of the bacteria kingdom such as a phylum, class, order, family, genus or species in the bacteria kingdom.

As used herein, the term "portion" refers to at least one percent, at least two percent, at least ten percent, at least twenty percent, at least thirty percent, at least fifty percent, as least seventy-five percent, at least ninety percent, or at least 99 percent of a set. Thus, in a nonlimiting example, at least a portion of a plurality of objects means at least one percent, at least two percent, at least ten percent, at least twenty percent, at least thirty percent, at least fifty percent, as least seventy-five percent, at least ninety percent, or at least 99 percent of the objects in the plurality.

As used herein, the term "Staphylococcaceae" refers to a family of bacteria in the Bacillales order that includes, but is not limited to, the *Staphylococcus aureus*, *Staphylococcus caprae*, *Staphylococcus epidermidis*, *Staphylococcus haemolyticus*, *Staphylococcus hominis*, *Staphylococcus lugdunensis*, *Staphylococcus pettenkoferi*, *Staphylococcus saprophyticus*, *Staphylococcus warneri*, and *Staphylococcus xylosus* bacteria.

As used herein, the term "Streptococcus" refers to a genus of spherical Gram-positive bacteria, belonging to the phylum Firmicutes and the lactic acid bacteria group. Cellular division occurs along a single axis in these bacteria, and thus they grow in chains or pairs, hence the name - from Greek streptos, meaning easily bent or twisted, like a chain. This is contrasted with staphylococci, which divide along multiple axes and generate grape-like clusters of cells. Species of Streptococcus include, but are not limited to *S. agalactiae*, *S. bovis*, *S. mutans, S. pneumoniae*, *S. pyogenes, S. salivarius*, *S. sanguinis*, *S. suis*, *Streptococcus viridans*, and *Streptococcus uberis*.

As used herein, a "subject" is an animal, preferably a mammal, more preferably a non-human primate, and most preferably a human. The terms "subject", "individual" and "patient" are used interchangeably herein.

As used herein, the term "vessel" refers to any container that can hold a culture such as a blood culture. For instance, in one embodiment a vessel is a container having a side wall, a bottom wall, an open top end for receiving a culture to be contained within the container, where the container is formed from a material such as glass, clear plastic (*e.g.,* a cyclic olefin copolymer) having a transparency sufficient to visually observe turbidity in the sample, and where the is preferably resistant to heating at a temperature of at least 250°C. In some embodiments, the container has a wall thickness sufficient to withstand an internal pressure of at least 25 psi and a closure coupled to the open end of the container, where the culture is substantially free of contamination after storage in the vessel for an extended period of time under ambient conditions. Exemplary containers are described in United States Patent No. 6,432,697. In some embodiments, the extended period of time under ambient conditions is at least about one year at about 40°C. In some embodiments, the vessel further comprises a fluorescent sensor compound fixed to an inner surface of the container that, when exposed to oxygen, exhibits a reduction in fluorescent intensity upon exposure to a fluorescing light. In some embodiments, the container is substantially transparent to said fluorescing light. In some embodiments, the fluorescent sensor compound comprises at least one compound selected from the group consisting of tris-4,7-diphenyl-1,10-phenanthroline ruthenium (II) salts, tris-2,2'-bipyridyl ruthenium (II) salts, 9,10-diphenyl anthracene, and mixtures thereof. In some embodiments, a vessel is a Blood Culture BACTEC®LYTIC/10 Anaerobic/F culture vial, a BBL® SEPTI-CHEK® vial, a BBL® SEPTI-CHEK® blood culture bottle, a Becton Dickinson BACTEC® vial, a Plus Aerobic/F* and Plus Anaerobic/F* culture vial, a Becton Dickinson BACTEC® Standard/10 Aerobic/F culture vial, a Becton Dickinson BACTEC® Myco/F Lytic culture vial, a Becton Dickinson BACTEC® PEDS PLUS®/F culture vial, or a Becton Dickinson BACTEC® Standard Anaerobic/F culture vial (Becton Dickinson, Franklin Lakes, New Jersey).

## 5.2 Exemplary Apparatus

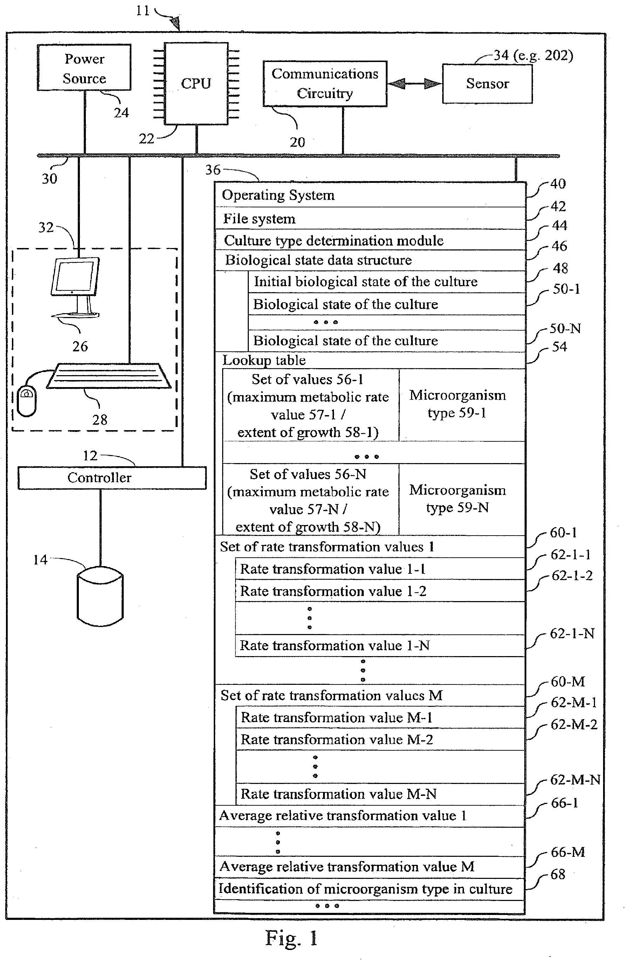

Figure 1 details an apparatus 11 for identifying a microorganism type in a culture in a vessel that comprises a processor and a memory, coupled to the processor. In some embodiments, the microorganism type identified by apparatus 11 or any of the methods of the present invention is a phylum within the bacteria kingdom. In some embodiments, the microorganism type identified by apparatus 11 or any of the methods of the present invention is a class within a phylum within the bacteria kingdom. In some embodiments, the microorganism type identified by apparatus 11 or any of the methods of the present invention is an order within a class within a phylum within the bacteria kingdom. In some embodiments, the microorganism type identified by apparatus 11 or any of the methods of the present invention is a family within an order within a class within a phylum within the bacteria kingdom. In some embodiments, the microorganism type identified by apparatus 11 or any of the methods of the present invention is a genus within a family within an order within a class within a phylum within the bacteria kingdom. In some embodiments, the microorganism type identified by apparatus 11 or any of the methods of the present invention is a species in a genus within a family within an order within a class within a phylum within the bacteria kingdom.

The processor and memory illustrated in Figure 1 can be, for example, part of an automated or semiautomated radiometric or nonradiometric microorganism culture system. The apparatus 11 preferably comprises:

- a central processing unit 22;
- optionally, a main non-volatile storage unit 14, for example a hard disk drive, for storing software and data, the storage unit 14 controlled by storage controller 12;
- a system memory 36, preferably high speed random-access memory (RAM), for storing system control programs, data, and application programs, comprising programs and data (optionally loaded from non-volatile storage unit 14); system memory 36 may also include read-only memory (ROM);
- a user interface 32, comprising one or more input devices (e.g., keyboard 28, a mouse) and a display 26 or other output device;
- a sensor 34 for taking a measurement of a biological state of a culture in a vessel;
- a network interface card 20 (communications circuitry) for connecting to the sensor 34;
- an internal bus 30 for interconnecting the aforementioned elements of the system; and
- a power source 24 to power the aforementioned elements.

Operation of central processing unit 22 is controlled primarily by operating system 40. Operating system 40 can be stored in system memory 36. In a typical implementation, system memory 36 also includes:

- a file system 42 for controlling access to the various files and data structures used by the present invention;
- a culture type determination module 44 for identifying a microorganism type in a culture in a vessel;
- a biological data structure 46 for storing an initial biological state 48 of the culture and a plurality of measurements of the biological state of the culture, where each measurement 50 in the plurality of measurements is taken at a different time point between a first (initial) time point and a second (final) time point;
- an optional lookup table 54 that comprises matches between (i) a plurality of sets of values, each set of values 56 in the plurality of sets of values comprising a maximum metabolic rate value 57 and an extent of growth 58, and (ii) a set of microorganism types, wherein, for each set of values 56 in the plurality of sets of values there is a corresponding microorganism type 59 in the set of microorganism types;
- sets of rate transformation values 60, where each set of rate transformation values comprises a plurality of rate transformation values 62, where each rate transformation value 62 is a first derivative of the normalization relative values associated with a predetermined fixed interval of time points;
- an average relative transformation value 66 for each set 60 of rate transformation values 60; and
- a data structure for storing an identification of a microorganism type 68 in a culture in a vessel.

As illustrated in Fig. 1, apparatus 11 can comprise data such as biological state data structure 46, optional lookup table 54, sets of rate transformation values 60, average relative transformation values 66, and an identification of microorganism type in culture 68. In some embodiments, memory 36 or data store 14 also stores a measure of central tendency of the average relative transformation values 66. The data described above can be in any form of data storage including, but not limited to, a flat file, a relational database (SQL), or an on-line analytical processing (OLAP) database (MDX and/or variants thereof). In some embodiments, such data structures are stored in a database that comprises a star schema that is not stored as a cube but has dimension tables that define hierarchy. Still further, in some embodiments, such data structures are stored in a database that has hierarchy that is not explicitly broken out in the underlying database or database schema (e.g., dimension tables that are not hierarchically arranged). In some embodiments, such data structures are stored in apparatus 11. In other embodiments, all or a portion of these data structures are hosted on (stored on) one or more computers that are addressable by apparatus 11 across an Internet / network that is not depicted in Figure 1. In some embodiments, all or a portion of one or more of the program modules depicted in apparatus 11 of Figure 1, such as culture type determination module 44 are, in fact, resident on a device (e.g., computer) other than apparatus 11 that is addressable by apparatus 11 across an Internet / network that is not depicted in Figure 1.

Apparatus 11 determines the metabolic activity of a culture by, for example, $CO_2$ concentration, $O_2$ concentration, pH, a rate of change in $CO_2$ concentration, a rate of change in $O_2$ concentration, or a rate of change in pH in the culture. From this metabolic activity determination, apparatus 11 can identify a microorganism type in the culture. In some embodiments, apparatus 11 accommodates a number of culture vessels and serves as an incubator, agitator, and detection system. These components of apparatus 11 are not depicted in Figure 1 because the nature of such components will vary widely depending on the exact configuration of apparatus 11. For instance, the number of culture vessels accommodated by apparatus can range from one vessel to more than 1000 vessels. There can be a sensor associated with each vessel in order to measure the biological state of the culture contained within the vessel. The sensor can be on any location of the vessel and there are a wide range of possible sensors that can be used.

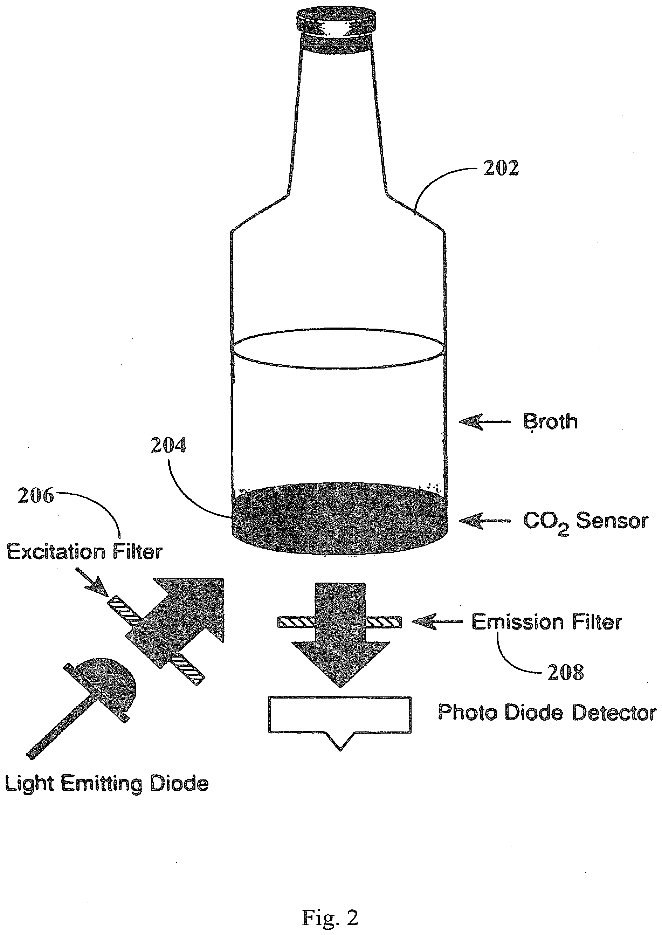

Figure 2 illustrates one exemplary sensor that is capable of measuring the biological state of a culture. In Figure 2, a $CO_2$ sensor 204 is bonded to the base of culture vessel 202 and overlaid with an amount of culture. $CO_2$ sensor 204 is impermeable to ions, medium components, and culture but is freely permeable to $CO_2$. Carbon dioxide produced by the cells in the culture diffuses into sensor 204 and dissolves in the water present in the sensor matrix, generating hydrogen ions. Increases in hydrogen ion concentration (decreases in pH) increase the fluorescence output of sensor 204, thereby changing the signal transmitted from excitation filter 206 to emission filter 208. Apparatus 11 takes repeated measurements of the signal penetrating emission filter 208 over time and uses this data to identify a microorganism type in the culture using the algorithms disclosed herein.

In some embodiments, apparatus 11 is an incubator, shaker, and fluorescence detector that will hold between 1 and 1000 culture vessels (e.g., 96, 240 or 384 culture vessels). In some embodiments, the vessels are arranged in racks (e.g., circular or linear racks), each of which has a number of vessel stations. For example, in one specific embodiment, apparatus 11 will hold 240 culture vessels arranged in six racks, where each rack has 40 vessel stations. In some embodiments, each vessel station in apparatus 11 contains a light-emitting diode and a photo diode detector with appropriate excitation and emission filters (*e.g.,* as illustrated in Figure 2). In some embodiments, the culture vessels are rocked and heated at 35 $\pm$ 1°C.

### 5.3 Exemplary Methods

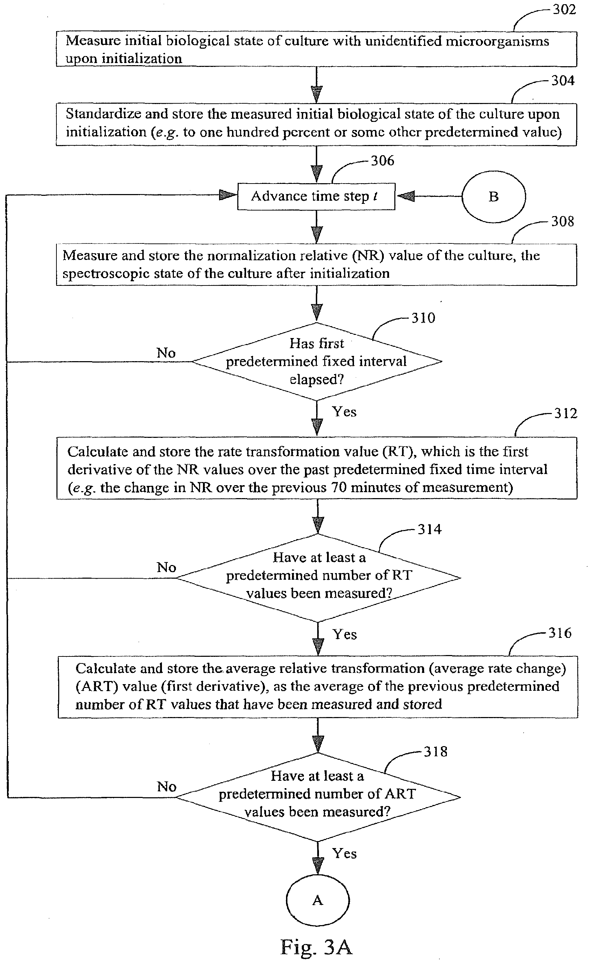

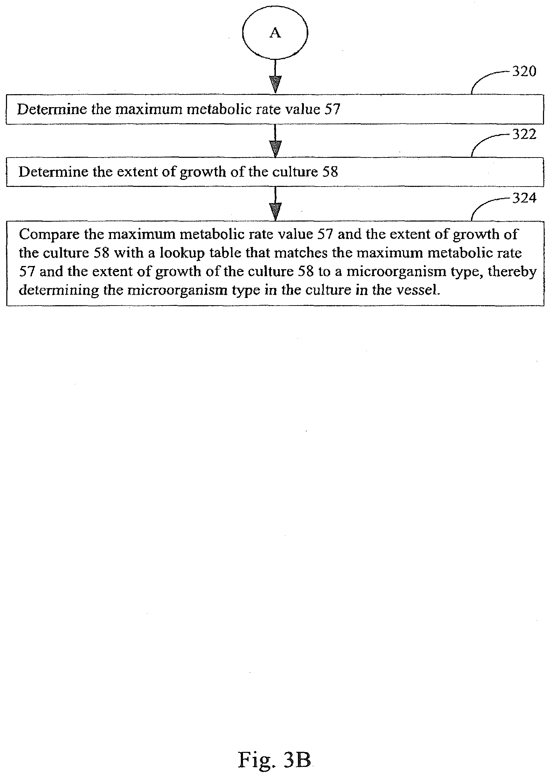

Now that an exemplary apparatus in accordance with the present invention has been described, exemplary methods in accordance with the present invention will be detailed. In some embodiments, such methods can be implemented by culture type determination module 44 of Figure 1. Referring to step 302 of Figure 3, an initial biological state of the culture is taken. For example, referring to Figure 2, in some embodiments, an initial read of detector 204 is made to determine the $CO_2$ concentration in the sensor. In alternative embodiments, an initial $O_2$ concentration, pH, or other indicia of the biological state of the culture is read (measured) in step 302. In some embodiments, the initial biological state of the culture is determined by a fluorescence output of a sensor (*e.g.*, sensor 204) that is in contact with the culture. In some embodiments, the amount of fluorescence output of the sensor is affected by $CO_2$ concentration in the manner described above in conjunction with Figure 2. In some embodiments, the amount of fluorescence output of the sensor is affected by $O_2$ concentration, pH, or some other indicia of metabolic state known in the art. In general, any physical observable that is indicative of the metabolic rate of the culture can be measured and stored as the initial state. In some embodiments, this physical observable is the accumulation of molecular products (an example being lipopolysaccharide with Gram negative bacteria), non-molecular physical / chemical changes to the environment related to growth (pressure changes), and/or the production of carbon dioxide or other metabolites that accumulate or the consumption of substrate such as oxygen) or the accumulation of cell material.

In some embodiments, an initial biological state of the blood culture is taken in step 302 using colorimetric means, fluorometric means, nephelometric means, or infrared means. Examples of colorimetric means include, but are not limited to, the use of the colorimetric redox indicators such as resazurine/methylene blue or tetrazolium chloride, or the of p-iodonitrotetrazolium violet compound as disclosed in United States Patent No. 6,617,127. Another example of colorimetric means includes the colormetric assay used in Oberoi et al. 2004, "Comparison of rapid colorimetric method with conventional method in the isolation of mycobacterium tuberculosis," Indian J Med Microbiol 22:44-46. In Oberoi *et al*., a MB/Bact240 system (Organon Teknika) is loaded with culture vessels. The working principle of this system is based on mycobacterial growth detection by a colorimetric sensor. If the organisms are present, $CO_2$ is produced as the organism metabolizes the substrate glycerol. The color of the gas permeable sensor at the bottom of each culture vessel results in increase of reflectance in the unit, which is monitored by the system using infrared rays. Examples of colorimetric means further include any monitoring of the change in a sensor composition color due to a change in gas composition, such as $CO_2$ concentration, in a vessel resulting from microorganism metabolism.

Examples of fluorometric and colorimetric means are disclosed in United States Patent No. 6,096,272, which discloses an instrument system in which a rotating carousel is provided for incubation and indexing, and in which there are multiple light sources each emitting different wavelength light for colorimetric and fluorometric detection. As used herein nephelometric means refers to the measurement of culture turbidity using a nephelometer. A nephelometer is an instrument for measuring suspended particulates in a liquid or gas colloid. It does so by employing a light beam (source beam) and a light detector set to one side (usually 90°) of the source beam. Particle density is then a function of the light reflected into the detector from the particles. To some extent, how much light reflects for a given density of particles is dependent upon properties of the particles such as their shape, color, and reflectivity. Therefore, establishing a working correlation between turbidity and suspended solids (a more useful, but typically more difficult quantification of particulates) must be established independently for each situation.

As used herein, an infrared means for measuring a biological state of a blood culture is any infrared microorganism detection system or method known in the art including, but not limited to, those disclosed United States Patent No. 4,889,992, as well as PCT publication number WO/2006071800.

In some embodiments, the vessel 202 holding the culture comprises a sensor composition 204 in fluid communication with the culture. The sensor composition 204 comprises a luminescent compound that exhibits a change in luminescent property, when irradiated with light containing wavelengths that cause the luminescent compound to luminesce, upon exposure to oxygen. The presence of the sensor composition 204 is non-destructive to the culture. In such embodiments, the measuring step 302 (and each instance of the measuring step 308) comprises irradiating the sensor composition 202 with light containing wavelengths that cause the luminescent compound to luminesce and observing the luminescent light intensity from the luminescent compound while irradiating the sensor composition with the light. In some embodiments, the luminescent compound is contained within a matrix that is relatively impermeable to water and non-gaseous solutes, but which has a high permeability to oxygen. In some embodiments, the matrix comprises rubber or plastic. More details of sensors in accordance with this embodiment of the present invention are disclosed in United States Patent No. 6,900,030.

In step 304, the measured initial biological state of the culture upon initialization from step 302 is standardized and stored as the initial biological state of the culture 48 (*e.g.* to one hundred percent or some other predetermined value). This initial biological state, stored as data element 48 in Figure 1, serves as a reference value against subsequent measurements of the biological state of the culture. In some embodiments, step 304 is not performed and the absolute measurements of step 302 are used in the algorithms disclosed herein.

Apparatus 11 incubates the culture for a predetermined period of time after the initial biological state measurement is taken. Then, after the predetermined period of time has elapsed, apparatus 11 makes another measurement of the biological state of the culture. This process is illustrated by steps 306 and 308 in Figure 3. In Figure 3A, the process is shown as advancing to time step $t$ in step 306. The biological state during the time period in step 306 in which apparatus waits for time to advance by time step $t$ is not used in subsequent processing steps to ascertain the microorganism type in the culture. In step 308, once time has advanced by time step $t$, a measurement of the biological state of the culture in the vessel is again taken in the same manner that the initial measurement of the biological state was taken (*e.g.*, using the device depicted in Figure 2). In some embodiments, the predetermined period of time (the duration of time step $t$) is ten minutes. In some embodiments, the predetermined period of time (the duration of time step $t$) is a period of time that is less than 5 minutes, a period of time that is less than 10 minutes, a period of time that is less than 15 minutes, a period of time that is less than 20 minutes, a period of time in the range between 1 minute and 30 minutes, a period of time in the range between 1 minute and 20 minutes, a period of time in the range between 5 minute and 15 minutes, or a period of time that is greater than 5 minutes. The measurement of the biological state of the culture in the vessel taken in step 308 is converted to a normalization relative value by standardizing the measurement of step 308 against the initial measurement of step 302 in embodiments where the initial measurement of step 302 is used for normalization. In one embodiment, the measurement of the biological state of the culture in the vessel taken in step 308 is converted to a normalization relative value by taking the ratio of the measurement of step 308 against the initial measurement of step 302. In some embodiments, this computed normalization relative value is stored as a data element 50 in Figure 1. In some embodiments, the measurement of the biological state measured in step 308 is stored as a data element 50 in Figure 1 and the normalization relative value corresponding to the measurement of the biological state measured in step 308 is computed as needed in subsequent processing steps.

In step 310 a determination is made as to whether a first predetermined fixed time interval has elapsed. For example, in some embodiments the predetermined fixed time interval is seventy minutes. In this example, if the time step $t$ of step 306 is 10 minutes, then it would require time step $t$ to have advanced seven times before condition 310-Yes is achieved. In some embodiments, the predetermined fixed interval of time is a duration of time that is between five minutes and five hours, a duration of time that is between twenty minutes and ten hours, a duration of time that is between twenty minutes and two hours, a duration of time that is between thirty minutes and ninety minutes, a duration of time that is less than 24 hours, or a duration of time that is more than 24 hours. When the first predetermined fixed interval of time has elapsed (310-Yes), process control passes on to step 312 where additional steps of the algorithm are performed. When the first predetermined fixed interval of time has not elapsed (310-No), process control passes back to step 306 where the algorithm waits for time to advance by the amount of time $t$ prior to once again taking a measurement of the biological state of the culture in a new instance of step 308.

The net result of steps 306 through 310 is that a plurality of measurements of a biological state of the culture in the vessel are taken and that each measurement in the plurality of measurements is at a different time point between a first (initial) time point and a terminating (final) time point. Further, in typical embodiments where time step $t$ is the same amount in each instance of step 306, the measurements in the plurality of measurements are each taken of the culture at a periodic interval. In some embodiments, the periodic interval is an amount of time between one minute and twenty minutes, an amount of time between five minutes and fifteen minutes, an amount of time between thirty seconds and five hours, or an amount of time that is greater than one minute.

When a predetermined fixed interval has elapsed (310-Yes), a first derivative of the normalization relative values in the respective predetermined fixed interval (or absolute values from step 302 in the respective predetermined fixed interval in embodiments in which normalization is not performed) is computed in step 312, thereby forming a rate transformation value 62. In other words, the change in the normalization relative values during the predetermined fixed interval is determined in step 312. Note that rate transformation values are the first derivative of normalization relative values in embodiments where measurement data is normalized and rate transformation values are the first derivative of absolute measurements from step 302 in embodiments where measurement data is not normalized. In some embodiments, the predetermined fixed interval of time over which the first derivative is computed is all measurements in an immediately preceding period of time that is between twenty minutes and two hours. For example, in some embodiments the predetermined fixed interval of time of step 310 is seventy minutes and, in step 312, the rate of change across all of the normalization relative values of measurements in this seventy minute time interval (the past 70 minutes) is determined in step 312 and stored as the rate transformation value 62. In some embodiments, the predetermined fixed interval of time over which the first derivative is computed (time window) is all measurements in an immediately preceding period of time that is between five minutes and twenty hours, between thirty minutes and ten hours, between twenty minutes and two hours, between twenty minutes and ten hours, or between thirty minutes and ninety minutes.

In step 314 a determination is made as to whether a predetermined number of rate transformation values have been measured since the last time condition 314-Yes was reached. If so (314-Yes), process control passes on to step 316. If not (314-No), process control returns back to step 306 where process control waits until time step *t* has elapsed before continuing to step 308 where the normalization relative value of the culture is once again calculated. Each condition (314-Yes) marks the completion of a set 60 of rate transformation values 62. For example, in some embodiments, condition 314-Yes is achieved when seven new rate transformation values 62 have been measured. In this example, a set 60 of rate transformation values comprises or consists of the seven rate transformation values 62. In some embodiments, each set 60 of rate transformation values 62 comprises or consists of between four and twenty contiguous rate transformation values. Contiguous rate transformation values 62 are rate transformation values in the same set 60. Such rate transformation values 62 are, for example, calculated and stored in successive instances of step 312. In some embodiments, each set 60 of rate transformation values 62 in the plurality of rate transformation values comprises or consists of between five and fifteen contiguous rate transformation values 62, between one and one hundred contiguous rate transformation values 62, between five and one fifteen contiguous rate transformation values 62, more than five rate transformation values 62, or less than ten rate transformation values 62.

When condition 314-Yes is achieved, step 316 is run. In step 316, an average relative transformation (average rate of change) value 66 is computed from the newly formed set 60 of rate transformation values 62. Thus, for each set 60 of rate transformation values 62, there is an average relative transformation value 66. In some embodiments, an average relative transformation (average rate of change) value 66 is computed from the newly formed set 60 of rate transformation values 62 by taking a measure of central tendency of the rate transformation values 62 in the newly formed set 60 of rate transformation values 62. In some embodiments, this measure of central tendency is a geometric mean, an arithmetic mean, a median, or a mode of all or a portion of the rate transformation values 62 in the newly formed set 60 of rate transformation values 62.

In step 318, a determination is made as to whether a predetermined point in the protocol has been reached. This predetermined point is a final time point, also known as an end point or second time point. In some embodiments, the second time point is reached (318-Yes) one or more hours, two or more hours, ten or more hours, between three hours and one hundred hours, or less than twenty hours after the initial measurement in step 302 was taken. In some embodiments, the second time point is reached (318-Yes) when between 10 and 50,000, between 100 and 10,000, or 150 and 5,000, more than 10, more than fifty, or more than 100 measurements of the biological state of the culture in the vessel have been made in instances of step 308. If the predetermined point in the protocol has not been reached (318-No), then process control returns to step 306 where the process control waits for time step *t* to advance before initiating another instance of step 308 in which the biological state of the culture is again measured and used to calculate a normalization relative value. If the predetermined point in the protocol has been reached (318-Yes), process control passes to step 320.

In step 320, the maximum metabolic rate value 57 achieved for the culture is determined. In some embodiments, the maximum metabolic rate 57 is deemed to be a maximum average relative transformation value 66 calculated in any instance of step 316 for the culture. For example, if the maximum average relative transformation value 66 ever calculated for the culture during an instance of step 316 is 250, then the maximum metabolic rate value 57 for the culture will be deemed to be 250.

In step 322, the extent of growth 58 of the culture is determined. In some embodiments, the extent of growth (EG) 58 is determined by the equation:

$$EG = NR_{after\_growth} - NR_{minimum\_growth}. \qquad \text{Eq. 1}$$

In some embodiments, $NR_{after\_growth}$ of Eq. 1 is a normalization relative value in the plurality of normalization relative values that was used in the calculation of the maximum average relative transformation value 66 ever achieved for the culture. Thus, if normalization relative values 135 through 144 were used to compute the maximum average relative transformation value 66, then $NR_{after\_growth}$ would be one of the normalization relative values in the set of normalization relative values {135, ..., 144}.

In some embodiments, $NR_{after\_growth}$ of Eq. 1 is a normalization relative value in the plurality of normalization relative values that was used in the calculation of the average relative transformation value 66 following the maximum average relative transformation value 66 ever achieved for the culture. Thus, if normalization relative values 145 through 154 were used to compute the average relative transformation value 66 following the maximum average relative transformation value 66, then $NR_{after\_growth}$ would be one of the normalization relative values in the set of normalization relative values {145, ..., 154}.

In some embodiments, $NR_{after\_growth}$ of Eq. 1 is a normalization relative value in the plurality of normalization relative values that was used in the calculation of the average relative transformation value 66 preceding the maximum average relative transformation value 66 ever achieved for the culture. Thus, if normalization relative values 125 through 134 were used to compute the average relative transformation value 66 immediately preceding the maximum average relative transformation value 66, then $NR_{after\_growth}$ would be one of the normalization relative values in the set of normalization relative values {125, ..., 134}.

In some embodiments, $NR_{after\_growth}$ of Eq. 1 is a measure of central tendency of all or a portion of the normalization relative values that were used in the calculation of the maximum average relative transformation value 66 ever achieved for the culture. Thus, if normalization relative values 135 through 144 were used to compute the maximum average relative transformation value 66, then $NR_{after\_growth}$ would be a measure of central tendency (geometric mean, an arithmetic mean, a median, or a mode) of all or a portion of the normalization relative values in the set of normalization relative values {135, ..., 144}.

In some embodiments, $NR_{after\_growth}$ of Eq. 1 is a measure of central tendency of all or a portion of the normalization relative values that were used in the calculation of the average relative transformation value 66 following the maximum average relative transformation value 66 ever achieved for the culture. Thus, if normalization relative values 145 through 154 were used to compute the average relative transformation value 66 following the maximum average relative transformation value 66, then $NR_{after\_growth}$ would be a measure of central tendency (geometric mean, an arithmetic mean, a median, or a mode) of all or a portion of the normalization relative values in the set of normalization relative values {145, ..., 154}.

In some embodiments, $NR_{after\_growth}$ of Eq. 1 is a measure of central tendency of all or a portion of the normalization relative values that were used in the calculation of the average relative transformation value 66 preceding the maximum average relative transformation value 66 ever achieved for the culture. Thus, if normalization relative values 125 through 134 were used to compute the average relative transformation value 66 immediately preceding the maximum average relative transformation value 66, then $NR_{after\_growth}$ would be a measure of central tendency (geometric mean, an arithmetic mean, a median, or a mode) of all or a portion of the normalization relative values in the set of normalization relative values {125, ..., 134}.

In some embodiments, $NR_{minimum\_growth}$ is a normalization relative value in the plurality of normalization relative values that was used in the calculation of the first average relative transformation value 66 to achieve a threshold value. Thus, if normalization relative values 20 through 29 were used to compute the first average relative transformation value 66 to achieve a threshold value, then $NR_{minimum\_growth}$ would be one of the normalization relative values in the set of normalization relative values {20, ..., 29}.

In some embodiments, $NR_{minimum\_growth}$ is a measure of central tendency of the normalization relative values that were used in the calculation of the first average relative transformation value 66 to achieve a threshold value. Thus, if normalization relative values 20 through 29 were used to compute the first average relative transformation value 66 to achieve a threshold value, then $NR_{minimum\_growth}$ would be all or a portion of the normalization relative values in the set of normalization relative values {20, ..., 29}.

In some embodiments where Equation 1 is used to calculate extent of growth 58, the threshold value is, in nonlimiting examples, a value between 5 and 100, a value between 25 and 75, a value between 1 and 1000, or a value that is less than 50.

In some embodiments, the extent of growth (EG) 58 is determined by the equation:

$$EG = ARTmax * (time_{ARTmax} - time_{initial}) \qquad Eq.\ 2$$

where ARTmax is a maximum average relative transformation value 66 achieved for the culture and $time_{ARTmax}$ is a duration of time between (a) the initial time point when the biological state of the culture was measured in time step 302 and (b) a time point when a normalization relative value used in the calculation of (i) the first average relative transformation value following the maximum average relative transformation value, (ii) the maximum average relative transformation value, or (iii) the first average relative transformation value preceding the maximum average relative transformation value determined for the culture by instances of step 316 was measured. Further, $time_{initial}$ is a duration of time between (i) the initial time point when the biological state of the culture was measured in time step 302 and (ii) a time point when a normalization relative value, used in the calculation of the first average relative transformation value to achieve a threshold value, was measured.

In some embodiments where Equation 2 is used to calculate extent of growth 58, the threshold value is, in nonlimiting examples, a value between 5 and 100, a value between 25 and 75, a value between 1 and 1000, or a value that is less than 50.

In some embodiments, the extent of growth (EG) 58 is determined by the equation:

$$EG = [ARTmax * (time_{ARTmax} - time_{initial})] / time_{initial} \qquad Eq. \ 3$$

where the values for ARTmax, time$_{ARTmax}$, and time$_{initial}$ are as given for Equation 2. In some embodiments where Equation 3 is used to calculate extent of growth 58, the threshold value is, in nonlimiting examples, a value between 5 and 100, a value between 25 and 75, a value between 1 and 1000, or a value that is less than 50.

The values ARTmax, time$_{ARTmax}$, time$_{initial}$ ,NR$_{after\_growth}$, and NR$_{minimum\_growth}$ as used in Equations 1, 2, or 3 can be individually multiplied by any real number provided that the same formalism is used when constructing reference extent of growth values 58 for optional lookup table 54. More generally, any of Equations 1, 2, or 3 can contain additional mathematical operations, both linear and nonlinear, and still be used to compute the extent of growth 58 provided that the same mathematical operations are used when constructing reference extent of growth values 58 for lookup table 54. Reference extent of growth values 58, and maximum metabolic rate value 57 are computed for lookup table 54 using cultures containing microorganism of known type.

In step 324, the maximum metabolic rate value 57 for the culture, determined in step 320, and the extent of growth 58 of the culture, determined in step 322, are used to determined the identity of the infecting microorganisms. In some embodiments of step 342, the maximum metabolic rate value 57 for the culture, determined in step 320, and the extent of growth 58 of the culture, determined in step 322, are compared with the values in lookup table 54 that matches the maximum metabolic rate 57 and the extent of growth 58 to a microorganism type 59, thereby determining the microorganism type in the culture in the vessel. To illustrate, consider the case in which the maximum metabolic rate value 57 for the culture is "200" and the extent of growth 58 of the culture is "450" and the values lookup table 54 has the following values:

| Maximum metabolic rate 56 | Extent of growth 58 | Microorganism type 59 |
| --- | --- | --- |
| 150 | 400 | X |
| 200 | 455 | Y |
| 254 | 502 | Z |

In this example, the microorganism type 59 for the culture will be deemed to be type Y because the values (200, 455) in the lookup table 54 are the values that most closely match the observed values for maximum metabolic rate and extent of growth (200, 450). This example illustrates a preferred embodiment of the present invention in which the set of values 56 (maximum metabolic rate 57, extent of growth 58) in lookup table 54 that mostly closely matches the observed maximum metabolic rate 57 and extent of growth 58 for a test culture are deemed to be the matching set of values and thus the test culture is deemed to be the microorganism type 59 in the lookup table 54 that corresponds to this matching set of values. In some embodiments, a lookup table is not used. In such embodiments, the maximum metabolic rate value 57 for the culture, determined in step 320, and the extent of growth 58 of the culture, determined in step 322 are used in one or more trained classifiers or other forms of equations (e.g., regression equations) that are capable of identifying microorganism based upon maximum metabolic rate value 57 and the extent of growth 58 for the culture.

In some embodiments, step 324 identifies the microorganism type 59 in the culture in the vessel as (i) a bacterium in the Enterobacteriaceae family or (ii) a bacterium not in the Enterobacteriaceae family based upon the maximum metabolic rate 57 and the extent of growth 58 of the test culture. For instance, in some embodiments, this is done by comparing the maximum metabolic rate 57 and the extent of growth 58 of the test culture with value in the lookup table 54. In other embodiments, this is done by used the maximum metabolic rate 57 and the extent of growth 58 in one or more trained classifiers and/or other forms of equations that take into accounts values of maximum metabolic rate 57 and the extent of growth 58 of known microorganism in culture. In some embodiments, step 324 identifies the microorganism type 59 as bacteria based upon the maximum metabolic rate 57 and the extent of growth 58 of the test culture. In some embodiments, step 324 identifies the microorganism type 59 as bacteria based upon the comparing of the maximum metabolic rate 57 and the extent of growth 58 of the test culture with values in the lookup table 54. In some embodiments, step 324 identifies the microorganism type as (i) Enterobacteriacea, (ii) Staphylococcaceae, (iii) Streptococcus, or (iv) Acinetobacter based the maximum metabolic rate 56 and the extent of growth 58 of the test culture. In some embodiments, step 324 identifies the microorganism type as (i) Enterobacteriacea, (ii) Staphylococcaceae, (iii) Streptococcus, or (iv) Acinetobacter based upon comparing the maximum metabolic rate 56 and the extent of growth 58 of the test culture with values in the lookup table 54. In some embodiments, step 324 identifies the microorganism type 59 as a single genera of the Enterobacteriaceae selected from the group consisting of *Alishewanella*, *Alterococcus*, *Aquamonas*, *Aranicola*, *Arsenophonus*, *Azotivirga*, *Blochmannia*, *Brenneria*, *Buchnera*, *Budvicia*, *Buttiauxella*, *Cedecea*, *Citrobacter*, *Dickeya*, *Edwardsiella*, *Enterobacter*, *Erwinia*, *Escherichia*, *Ewingella*, *Griimontella*, *Hafnia*, *Klebsiella*, *Kluyvera*, *Leclercia*, *Leminorella*, *Moellerella*, *Morganella*, *Obesumbacterium*, *Pantoea*, *Pectobacterium*, *Candidatus Phlomobacter*, *Photorhabdus*, *Plesiomonas*, *Pragia*, *Proteus*, *Providencia*, *Rahnella*, *Raoultella*, *Salmonella*, *Samsonia*, *Serratia*, *Shigella*, *Sodalis*, *Tatumella, Trabulsiella*, *Wigglesworthia*, *Xenorhabdus*, *Yersinia*, and *Yokenella* based upon the maximum metabolic rate 56 and the extent of growth 58 of the test culture. In some embodiments, step 324 identifies the microorganism type 59 as a single genera of the Enterobacteriaceae selected from the group consisting of *Alishewanella*, *Alterococcus*, *Aquamonas*, *Aranicola*, *Arsenophonus*, *Azotivirga*, *Blochmannia*, *Brenneria*, *Buchnera*, *Budvicia*, *Buttiauxella*, *Cedecea*, *Citrobacter*, *Dickeya*, *Edwardsiella*, *Enterobacter*, *Erwinia*, *Escherichia*, *Ewingella*, *Griimontella*, *Hafnia*, *Klebsiella*, *Kluyvera*, *Leclercia*, *Leminorella*, *Moellerella*, *Morganella*, *Obesumbacterium*, *Pantoea*, *Pectobacterium*, *Candidatus Phlomobacter*, *Photorhabdus*, *Plesiomonas*, *Pragia*, *Proteus*, *Providencia*, *Rahnella*, *Raoultella*, *Salmonella*, *Samsonia*, *Serratia*, *Shigella*, *Sodalis*, *Tatumella, Trabulsiella*, *Wigglesworthia*, *Xenorhabdus*, *Yersinia*, and *Yokenella* based upon comparing the maximum metabolic rate 56 and the extent of growth 58 of the test culture with values in the lookup table 54.

In some embodiments, step 324 identifies the microorganism type 59 as a single species of Staphylococcaceae selected from the group consisting of *Staphylococcus aureus*, *Staphylococcus caprae, Staphylococcus epidermidis*, *Staphylococcus haemolyticus*, *Staphylococcus hominis*, *Staphylococcus lugdunensis*, *Staphylococcus pettenkoferi*, *Staphylococcus saprophyticus*, *Staphylococcus warneri*, and *Staphylococcus xylosus* bacteria. In some embodiments, the determining step identifies the microorganism type as a single species of Streptococcus selected from the group consisting of S. *agalactiae*, *S. bovis*, *S. mutans, S. pneumoniae*, *S. pyogenes, S. salivarius*, *S. sanguinis*, *S. suis*, *Streptococcus viridans*, and *Streptococcus uberis* based upon the maximum metabolic rate 56 and the extent of growth 58 of the test culture. For example, in some embodiments, the step 324 identifies the microorganism type as a single species of Streptococcus selected from the group consisting of S. *agalactiae*, *S. bovis*, *S. mutans, S. pneumoniae*, *S. pyogenes, S. salivarius*, *S. sanguinis*, *S. suis*, *Streptococcus viridans*, and *Streptococcus uberis* based upon comparing the maximum metabolic rate 56 and the extent of growth 58 of the test culture with values in the lookup table 54.

In some embodiments, step 324 identifies the microorganism type as aerobic based upon the maximum metabolic rate 56 and the extent of growth 58. In some embodiments, step 324 identifies the microorganism type 59 as anaerobic based upon the maximum metabolic rate 57 and extent of growth 58 of the test culture. In some embodiments, step 324 identifies the microorganism type as aerobic based upon comparing of the maximum metabolic rate 56 and the extent of growth 58 with values in the lookup table 54. In some embodiments, step 324 identifies the microorganism type 59 as anaerobic based upon comparing the maximum metabolic rate 57 and extent of growth 58 of the test culture with values in the lookup table 54.

In some embodiments, the method further comprises outputting an identification of microorganism type in culture 68 to a user interface device (*e.g.,* 32), a monitor (*e.g.,* 26), a computer-readable storage medium (*e.g.,* 14 or 36), a computer-readable memory (*e.g.,* 14 or 36), or a local or remote computer system. In some embodiments an identification of microorganism type in culture 68 is displayed. As used herein, the term local computer system means a computer system that is directly connected to apparatus 11. As used herein, the term remote computer system means a computer system that is connected to apparatus 11 by a network such as the Internet.

## 5.4 Exemplary Computer Program Products and Computers

The present invention can be implemented as a computer program product that comprises a computer program mechanism embedded in a computer-readable storage medium. Further, any of the methods of the present invention can be implemented in one or more computers. Further still, any of the methods of the present invention can be implemented in one or more computer program products. Some embodiments of the present invention provide a computer program product that encodes any or all of the methods disclosed herein. Such methods can be stored on a CD-ROM, DVD, magnetic disk storage product, or any other computer-readable data or program storage product. Such methods can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. Such methods encoded in the computer program product can also be distributed electronically, via the Internet or otherwise.

Some embodiments of the present disclosure provide a computer program product that contains any or all of the program modules and data structures shown in Figure 1. These program modules can be stored on a CD-ROM, DVD, magnetic disk storage product, or any other computer-readable data or program storage product. The program modules can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. The software modules in the computer program product can also be distributed electronically, via the Internet or otherwise.

### 5.5 Kits

Some embodiments of this disclosure may also comprise a kit to perform any of the methods described herein. In a non-limiting example, vessels, culture for a sample, additional agents, and software for performing any combination of the methods disclosed herein may be comprised in a kit. The kits will thus comprise one or more of these reagents in suitable container means.

The components of the kits, other than the software, vessels, and the radiometric or nonradiometric system, may be packaged either in aqueous media or in lyophilized form. The suitable container means of the kits will generally include at least one vial, test tube, flask, bottle, syringe or other container means, into which a component may be placed, and preferably, suitably aliquoted. Where there is more than one component in the kit, the kit also will generally contain a second, third or other additional container into which the additional components may be separately placed. However, various combinations of components may be in a vial. The kits also will typically include a means for containing the reagent containers in close confinement for commercial sale. Such containers may include injection or blow-molded plastic containers into which the desired vials are retained.

### 6 EXAMPLE

A presumptive organism identification test has been developed for any culture system designed to culture a biological sample (hereinafter "culture") for the presence of unknown microorganisms with the intended use of determining the presence and identification of a microorganism in the culture. The principle is a culture is inoculated into one or more culture vials, the vials are inserted into an apparatus 11 and the apparatus monitors the vials for the presence of metabolic activity (the production of carbon dioxide or other metabolites that accumulate or the consumption of substrate such as oxygen) or the accumulation of cell material (by measuring the accumulation of microbial cells). The BACTEC® blood culture system is an example of such an apparatus 11. The BACTEC® blood culture system uses fluorescent sensors that report changes to the system when microbial metabolism occurs. The algorithm depicted in Figure 3 is then applied to the sequence of signal data. The algorithm was designed to recognize signal changes with time that were indicative of the presence of growing microorganisms. The user was notified when the system recognized evidence of growth (status change to a positive vial) and the vessel was then processed to determine the presence of an organism (Gram stain and subculture to a plated medium). The inventive algorithm utilized the differences in rate of growth and extent of growth to provide information about the microorganisms present and growing in the culture. This information, in conjunction with a lookup table that records similar information about known microorganisms, resulted in the identification of the microorganism type in the culture.

Data that was collected with the BACTEC® blood culture system is used as an example of the application of the inventive data transformation illustrated in Figure 3 and provided examples of the utility of the present invention. The BACTEC® system, as described above, uses fluorescent sensors to monitor the changes in metabolic activity within the culture through a stream of compensated fluorescence signal data that is collected at ten minute intervals from a sensor located inside the culture reagent. The data used in this example was collected from the BACTEC® instruments used either in internal seeded culture studies or collected during a clinical evaluation of the system. The data was sorted and collected into a database at Becton Dickinson that includes the identification of the vessel (by sequence and accession numbers), a record of the dates of inoculation, the amount of blood in the sample (it is a blood culture system) and the result with the identification of the microorganism found in the vessel (the organism identification was provided by the clinical site in the case of the external data). The algorithm illustrated in Figure 3 was applied subsequently for analysis. The utility of this information is as a presumptive identification into closely related types of organisms. In this example, microorganisms in clinical blood cultures were characterized into four different microorganism types: Enterobacteriaceae, (ii) Staphylococcaceae, (iii) Streptococcus, or (iv) Acinetobacter. Closely related species of microorganisms that are indicative of these general classifications of microorganisms separated together (*Escherichia coli* and *Klebsiella pneumoniae* separated together and *Staphylococcus aureus* and coagulase negative staphylococci separated together). In some instances, individual species of microorganisms could be identified with a high degree of confidence. The fundamental principle for utilizing these data for presumptive identification is that the type of metabolic processes these microorganisms possessed and the relative bioenergetics of these different types of microorganisms could be distinguished by monitoring a variety of rate functions associated with growth under defined conditions.

The inventive data transformations began with an initial normalization of the vessel signal to a specific output (its initial state upon entering the system), as described above in conjunction with steps 302 and 304 of Figure 3. All subsequent data was represented as a percentage of that initial signal, which has been standardized to 100 percent in these analyses, as outlined in steps 306 and 308 of Figure 3. Data measurements normalized by the initial signal were termed normalization relative values. Under ideal theoretical conditions, a normalization relative value of 125 means that microorganism metabolism caused the fluorescence measured by the BACTEC® sensor to increase by 25 percent relative to the initial measurement. The next value that was computed was the first derivative of the NR value as it changes with time as outlined in steps 310 and 312 of Figure 3. This value was the rate transformation (RT) value and the base RT value used in this example uses a periodicity limit of 70 minutes. Any given RT value represented the rate of percentage change of fluorescence signal over the seventy minutes prior to its calculation. The next value that was computed was the ART or average rate change value as outlined in steps 314 and 316 of Figure 3. This was calculated as the average of the previous 7 ART values that had been calculated and acted as a smoothing function of the RT value.

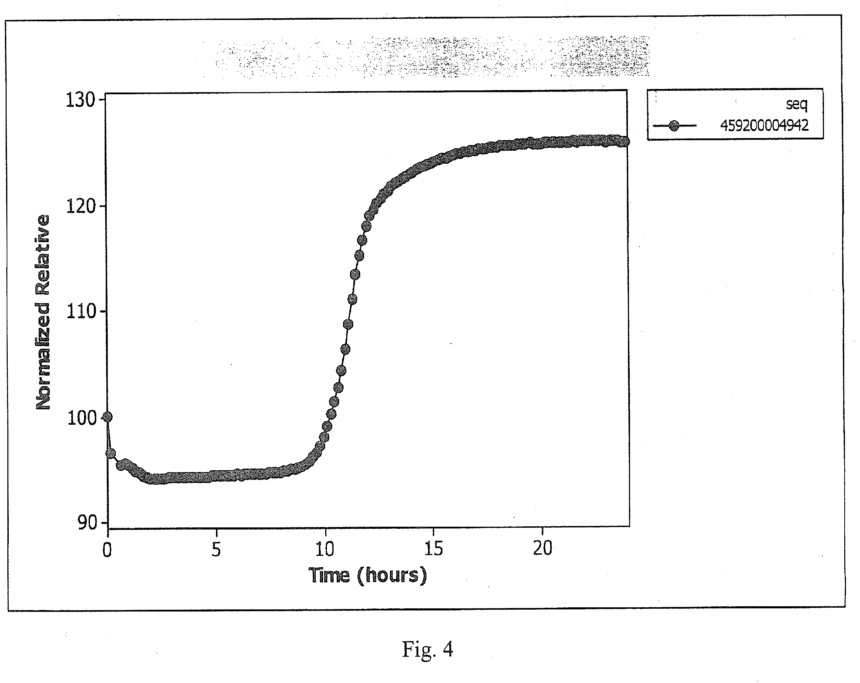

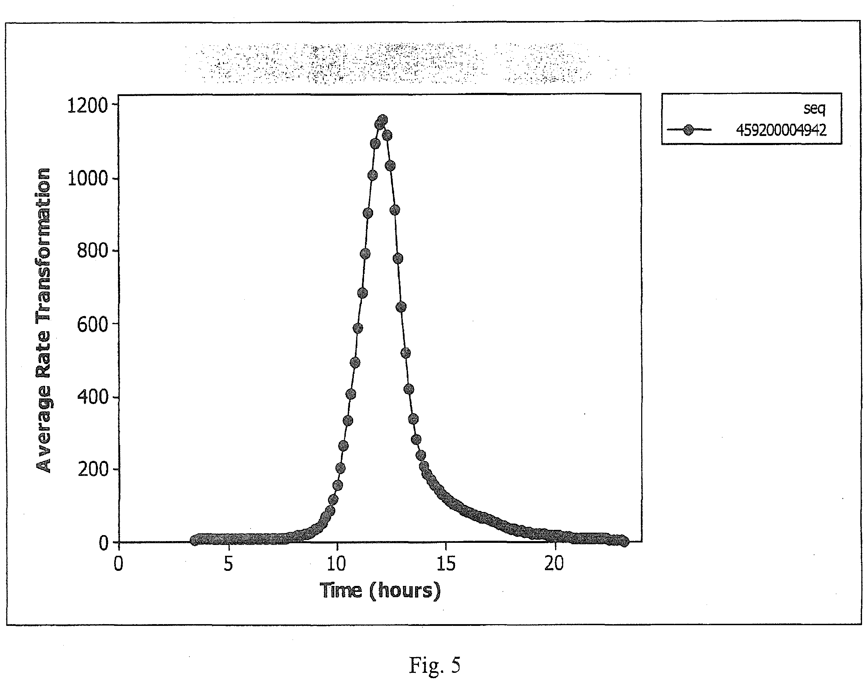

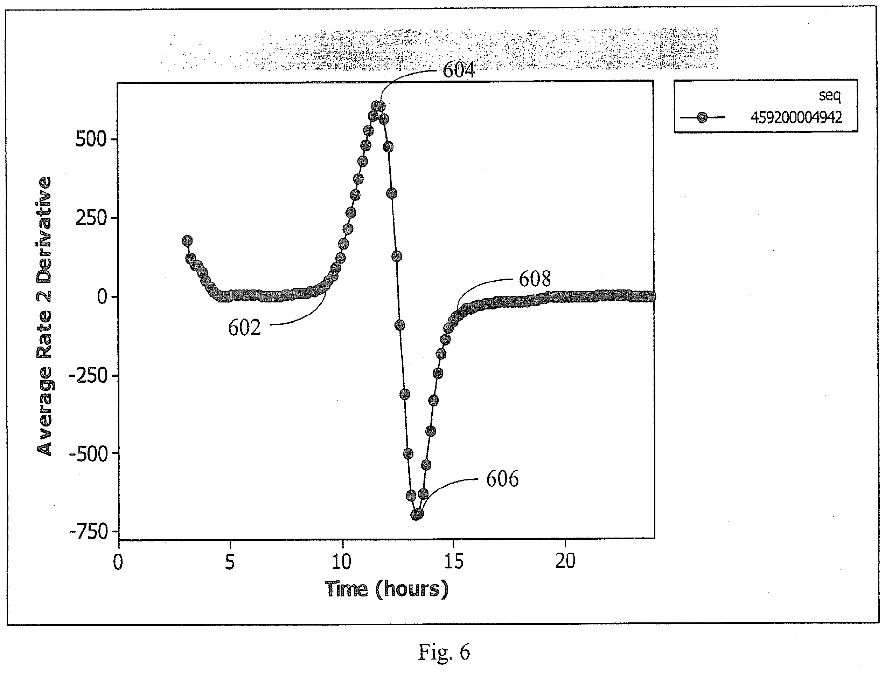

Examples of the parameters that were computed to determine the identity of a type of microorganism infecting a culture are presented in Figures 4, 5 and 6. An *Escherichia coli* culture was analyzed using these quantitative metrics (the normalized relative values 50, the rate transformation values 62, and the average relative transformation values 66. The culture contained three milliliters of human blood from a subject and was inoculated with a suspension of *E. coli* (55 CFU) and entered into a BACTEC® 9000 instrument. The identifier 4942 is unique identifies the culture that is reported in Figures 4, 5, and 6 and can be used to link the data for this culture to a research and development BACTEC® database. Figure 4 shows a plot of normalization relative values over time. The vessel was entered into the instrument and temperature affects related to equilibration of the vessel were observed for approximately the first hour. The signal stabilized and a background was observed to increase from 94 percent to 95 percent of the initial signal for the first hour (this rate was due to blood activity). In the normalization relative plot (Figure 4), growth was visible beginning at eight hours and proceeded until 15 hours with a final value NR value near 126. The plot of average relative transformation values 66 over time based on the average rate of change in rate transformation values 62 of Figure 4 over time is provided in Figure 5. Each average relative transformation (ART) value 66 is a measure of the average rate of change and the maximum ART for this culture was 1158 achieved at 12.8 hours into the culture. This represents this culture's averaged maximum achieved rate of sensor change over a one hour period. Figure 6 is the second derivative plot of normalization relative values 50 and shows the changes in rate with time. This is a graphical interpretation showing the following critical points: the point of initial acceleration 602 (movement from the null), the point of maximum acceleration 604 (the maxima), where acceleration reaches its maximum (crossing the null point), the maximum point of deceleration (the minima) 606, and the terminus of the growth curve 608 (where the rate change returns to null).

The growth features that used the above-identified data transformations (rate transformation values 60, average relative transformation value 66, and the normalization relative values) for presumptive identification of microorganism type are the maximum metabolic rate and extent of growth. In this example, the maximum metabolic rate (ARTmax) was defined as the maximum ART value 66 achieved (in this example, the maximum metabolic rate was 1158).

In this example, the extent of growth (EG) was determined as

$$\mathrm{EG} = \mathrm{NR}_{\mathrm{after\_growth}} - \mathrm{NR}_{\mathrm{minimum\_growth}}. \qquad \mathrm{Eq.\ 1}$$

which is the difference of the normalization relative (NR) value before growth ($NR_{minimum\_growth}$) and after growth ($NR_{after\_growth}$). To standardize the definition of before and after growth, the following formalism was used to determine points representing before growth ($NR_{minimum\_growth}$) and after growth ($NR_{after\_growth}$). $NR_{minimum\_growth}$ was defined as the point where growth was discernable from the background signal. The time point at which $NR_{minimum\_growth}$ was measured was referred to as $time_{initial}$. An ART value 66 of value 50 defined the initiation of growth (this time strongly correlates to the time to detection for most cultures in the BACTEC® system). A defined point after the maximum ART was used to define the point of $NR_{after\_growth}$ growth extent comparison. Of course, other formalisms could be used to determine points representing before and after growth for the purpose of determining extent of growth and all such formalisms are within the scope of the present invention. In *the E coli* example illustrated in Figure 4, the time to NR = 50 is 9.6 hours and this corresponds to an NR value of 95.7. So, $NR_{minimum\_growth}$ was 95.7. From Figure 5, it was seen that the time to the first point following the ART maximum, referred to in this example as $time_{ARTmax}$, was 12.3 hours. From Figure 4, 12.3 hours corresponds to an NR value = 119.5. So, $NR_{after\_growth}$ was 119.5. Therefore, the extent of growth determined for this culture was 119.5 - 95.7 which equals 23.8 NR (which equals percent change in signal over the defined period). Further, from Figure 5 it was seen that the maximum growth rate is 1158 ART. These values for maximum growth rate and extent of growth were compared to a lookup table 54 that contains reference sets of values 56 for maximum metabolic rate 57 and extent of growth 58. The microorganism type 59 that corresponded to the set of values 56 that most closely matched the experimentally determined values for maximum growth rate and extent of growth was deemed to be the microorganism type for the culture.

Another value that was used to determine extent of growth was:

$$EG = ARTmax * (time_{ARTmax} - time_{initial}) \qquad Eq.\ 2$$

in which ARTmax was multiplied by the time difference ($time_{ARTmax}$ - $time_{initial}$), where $time_{ARTmax}$ was as defined as the time to the first point following the ARTmax and $time_{initial}$ was defined as the point where NR achieved a value of 50. This value for extent of growth could be used in conjunction with or instead of the extent of growth computed using equation 1. For this example, extent of growth as defined by Equation 2 had a value of 3281.

In some embodiments, the extent of growth (EG) 58 was determined by the equation:

$$EG = [ARTmax * (time_{ARTmax} - time_{initial})] / time_{initial} \qquad Eq.\ 3$$

where the values for ARTmax, $time_{ARTmax}$, and $time_{initial}$ are as given for Equations 1 and 2. For this example, extent of growth as defined by Equation 3 had a value of 353.

Table 1 is a list of 96 clinically significant isolates that were detected in recent clinical evaluation of the BACTEC® system. The instrumented data was collected and sent to Becton Dickinson for data analysis. Table 1 contains the rate data including the key descriptor calculations mentioned above (*e.g.*, extent of growth, maximum metabolic rate). These data are representative of microorganisms that are routinely recovered in blood. In Table 1, column 1 is a unique culture identifier, column 2 is an indication of the microorganism identified in the culture (ENTFA1 is *Enterococcus faecalis*, ENTCFAA is *Enterococcus faecium,* STRSANGR is the *Streptococcus sanguinous* group, STRAHE is *Streptococcus alpha hemolytic,* STRAGA is *Streptococcus agalactiae,* ESCCOL is *Escherichia coli,* SAUR is *Staphylococcus aureus*, STACNEG is *Coagulase negative Staphylococcus,* PROTMIR is *Proteus mirabilis,* KLEPNEP is *Klebsiella pneumoniae*, NEIMEN is *Neiserria meningitidis,* and ACINBAU is *Acinetobacter baumanii*), column 3 is an initialization time in hours, column 4 is a value for the maximum ART value achieved by the culture, column 5 is the time to achieve the maximum ART value in hours, column 6 is the time it took to reach an ART value of 50 in hours, column 7 is the time it took to reach an ART value of 20 in hours, column 8 is difference between the time it took to the maximum ART value minus the time it took to reach an ART value of 50 in hours, column 9 is the ratio between the maximum ART value and the maximum rate transformation value, column 10 is the an ARTmaxInterval50 value which is calculated as ARTmax multiplied by the time difference between (i) the time it took to achieve ARTmax and (ii) the time it took to achieve an ART value of 50, and column 11 is and ARTmaxInterval50 divided by the time it took the culture to reach an ART value of 50 in hours (the value in column 10 divided by the time it took to achieve an ART value of 50 in hours).

| Sequence (Column 1) | Isolate (Column 2) | TTD (Column 3) | MaxART (Column 4) | Time MaxART (Column 5) | Time ART@50 (Column 6) | Time ART@20 (Column 7) | TimeMaxART - TimeART@50 (Column 8) | Ratio Mart_ TMAXA RT (Column 9) | Interval 50 (Column 10) | Int50_TT ART50 (Column 11) |
|---|---|---|---|---|---|---|---|---|---|---|
| 1 | ENTCFAI | 10.00 | 687.07 | 11.57 | 9.40 | 8.38 | 2.17 | 59.39 | 1489.02 | 158.39 |
| 2 | ENTCFAA | 14.10 | 566.33 | 16.35 | 14.02 | 13.35 | 2.33 | 34.63 | 1321.76 | 94.28 |
| 3 | ENTCFAA | 9.70 | 348.38 | 10.43 | 6.75 | 6.21 | 3.69 | 33.40 | 1284.06 | 190.34 |
| 4 | ENTCFAA | 10.80 | 741.87 | 13.22 | 10.88 | 10.21 | 2.34 | 56.13 | 1735.83 | 159.59 |
| 5 | ENTCFAA | 12.40 | 372.63 | 15.44 | 12.10 | 11.10 | 3.34 | 24.13 | 1245.81 | 102.97 |
| 6 | ENTCFAA | 28.20 | 250.28 | 31.74 | 27.54 | 26.37 | 4.20 | 7.89 | 1050.90 | 38.16 |
| 7 | ENTCFAA | 12.50 | 892.42 | 14.86 | 12.19 | 11.36 | 2.67 | 60.06 | 2380.26 | 195.23 |
| 8 | ENTCFAI | 10.60 | 889.70 | 12.69 | 10.52 | 9.69 | 2.17 | 70.09 | 1931.36 | 183.54 |
| 9 | STRSANGR | 11.30 | 727.77 | 12.67 | 10.67 | 10.34 | 2.00 | 57.42 | 1455.69 | 136.38 |
| 10 | STRANG | 21.10 | 560.87 | 24.10 | 21.27 | 20.60 | 2.83 | 25.99 | 1775.15 | 83.47 |
| 11 | STRAHE | 14.20 | 971.12 | 15.95 | 13.45 | 10.44 | 2.50 | 35.16 | 1402.62 | 104.28 |
| 12 | STRAGA | 7.80 | 1097.53 | 9.19 | 7.02 | 3.69 | 2.17 | 105.69 | 2104.90 | 299.80 |
| 13 | STRAGA | 8.80 | 1133.48 | 10.19 | 8.19 | 7.52 | 2.00 | 107.73 | 2195.94 | 268.19 |
| 14 | ESCCOL | 11.00 | 1065.62 | 12.76 | 10.18 | 8.84 | 2.58 | 88.82 | 2925.06 | 287.31 |
| 15 | ESCCOL | 9.10 | 1121.82 | 11.18 | 8.84 | 7.85 | 2.34 | 95.31 | 2494.40 | 282.14 |
| 16 | ESCCOL | 8.90 | 1325.20 | 10.93 | 8.26 | 7.76 | 2.67 | 102.68 | 2992.12 | 362.33 |
| 17 | ESCCOL | 11.20 | 975.38 | 12.73 | 10.10 | NC | 2.64 | 104.08 | 3494.02 | 346.11 |
| 18 | ESCCOL | 8.70 | 1246.27 | 10.26 | 7.92 | 7.59 | 2.33 | 95.08 | 2276.73 | 287.32 |
| 19 | ESCCOL | 9.30 | 1333.27 | 11.31 | 8.51 | NC | 2.80 | 110.24 | 3489.93 | 410.34 |
| 20 | ESCCOL | 10.60 | 1239.10 | 12.35 | 9.52 | 4.34 | 2.83 | 107.93 | 3778.35 | 396.93 |
| 21 | ESCCOL | 11.00 | 1084.38 | 12.90 | 10.22 | 9.72 | 2.68 | 96.06 | 3320.42 | 324.93 |
| 22 | ESCCOL | 11.30 | 1373.55 | 13.34 | 10.84 | 10.50 | 2.50 | 81.31 | 2711.82 | 250.28 |
| 23 | ESCCOL | 10.80 | 1299.52 | 11.90 | 8.72 | 8.39 | 3.18 | 115.43 | 4367.89 | 500.96 |
| 24 | ESCCOL | 7.10 | 1266.70 | 9.18 | 6.34 | 4.01 | 2.83 | 141.59 | 3682.71 | 580.50 |
| 25 | ESCCOL | 7.30 | 297.63 | 9.85 | 7.18 | 6.68 | 2.67 | 128.66 | 3378.54 | 470.68 |
| 26 | ESCCOL | 17.00 | 1159.62 | 20.56 | 16.72 | 13.38 | 3.84 | 14.48 | 1142.66 | 68.35 |
| 27 | ESCCOL | 10.10 | 892.88 | 12.94 | 9.93 | 8.77 | 3.00 | 89.65 | 3480.48 | 350.36 |
| 924028 | ESCCOL | 10.50 | 829.72 | 13.17 | 10.34 | 9.00 | 2.83 | 67.81 | 2530.06 | 244.81 |
| 29 | SAUR | 12.00 | 614.18 | 13.51 | 11.18 | NC | 2.33 | 61.40 | 1936.73 | 173.25 |
| 30 | SAUR | 13.80 | 676.02 | 16.51 | 13.85 | 13.01 | 2.67 | 37.19 | 1638.14 | 118.30 |
| 31 | SAUR | 21.80 | 988.80 | 23.81 | 21.48 | 15.12 | 2.33 | 28.39 | 1577.56 | 73.46 |

| Sequence (Column 1) | Isolate (Column 2) | TTD (Column 3) | MaxART (Column 4) | Time MaxART (Column 5) | Time ART@50 (Column 6) | Time ART@20 (Column 7) | TimeMaxART - TimeART@50 (Column 8) | Ratio Mart_ TMAXA RT (Column 9) | Interval 50 (Column 10) | Int50_TT ART50 (Column 11) |
|---|---|---|---|---|---|---|---|---|---|---|
| 32 | SAUR | 15.30 | 596.58 | 17.35 | 14.85 | 12.85 | 2.50 | 57.00 | 2472.59 | 166.54 |
| 33 | SAUR | 22.30 | 697.33 | 24.68 | 22.01 | 21.01 | 2.67 | 24.17 | 1590.54 | 72.25 |
| 34 | SAUR | 14.70 | 687.07 | 16.87 | 14.37 | 13.70 | 2.50 | 41.34 | 1743.88 | 121.36 |
| 35 | SAUR | 11.20 | 698.28 | 13.54 | 10.87 | 10.03 | 2.67 | 51.57 | 1866.50 | 171.74 |
| 36 | SAUR | 14.50 | 764.00 | 16.70 | 14.20 | 13.37 | 2.50 | 45.74 | 1910.61 | 134.52 |
| 37 | SAUR | 16.90 | 467.43 | 19.12 | 14.45 | 14.12 | 4.67 | 24.45 | 2182.01 | 150.97 |
| 38 | SAUR | 14.90 | 984.03 | 14.92 | 13.57 | 13.24 | 1.34 | 65.97 | 1319.78 | 97.23 |
| 39 | SAUR | 14.90 | 819.72 | 14.92 | 13.57 | NC | 1.34 | 54.96 | 1099.41 | 80.99 |
| 40 | SAUR | 13.40 | 864.43 | 15.29 | 12.76 | 11.75 | 2.53 | 56.55 | 2188.65 | 171.59 |
| 41 | SAUR | 16.60 | 790.62 | 18.70 | 16.35 | 14.85 | 2.35 | 42.28 | 1858.59 | 113.69 |
| 42 | SAUR | 21.30 | 651.02 | 23.03 | 20.51 | 18.18 | 2.51 | 28.27 | 1636.08 | 79.75 |
| 43 | SAUR | 16.00 | 799.97 | 18.03 | 15.68 | 13.85 | 2.35 | 44.36 | 1880.65 | 119.93 |
| 44 | SAUR | 17.50 | 698.57 | 20.57 | 17.18 | 16.18 | 3.38 | 33.97 | 2361.52 | 137.43 |
| 45 | SAUR | 22.20 | 811.25 | 24.28 | 22.11 | 21.28 | 2.17 | 33.42 | 1758.38 | 79.53 |
| 46 | SAUR | 28.10 | 1197.85 | 23.68 | 19.83 | 18.83 | 3.85 | 50.58 | 4613.04 | 232.64 |
| 47 | SAUR | 28.00 | 1084.03 | 22.34 | 19.16 | 18.49 | 3.18 | 48.52 | 3447.87 | 179.93 |
| 48 | SAUR | 18.50 | 1017.80 | 20.74 | 17.90 | 33.84 | 2.84 | 49.08 | 2891.37 | 161.56 |
| 49 | SAUR | 9.70 | 972.92 | 12.06 | 8.37 | NC | 3.69 | 80.67 | 3594.65 | 429.67 |
| 50 | SAUR | 35.10 | 624.30 | 39.81 | 35.48 | NC | 4.33 | 15.68 | 2706.22 | 76.28 |
| 51 | SAUR | 15.60 | 1003.82 | 17.64 | 15.12 | 13.45 | 2.52 | 56.92 | 2524.61 | 166.97 |
| 52 | SAUR | 14.20 | 1082.83 | 16.28 | 13.61 | 22.96 | 2.67 | 66.50 | 2888.23 | 212.15 |
| 53 | SAUR | 31.60 | 941.75 | 26.96 | 23.26 | 23.09 | 3.70 | 34.93 | 3485.51 | 149.86 |
| 54 | SAUR | 11.80 | 777.80 | 15.36 | 11.86 | 11.02 | 3.50 | 50.64 | 2723.16 | 229.65 |
| 55 | SAUR | 14.00 | 669.53 | 18.75 | 14.69 | 13.69 | 4.06 | 35.70 | 2718.49 | 185.03 |
| 56 | SAUR | 18.60 | 643.83 | 20.50 | 17.66 | 12.31 | 2.83 | 31.41 | 1824.68 | 103.30 |
| 57 | SAUR | 19.80 | 657.62 | 22.48 | 19.31 | 18.31 | 3.17 | 29.25 | 2085.25 | 108.00 |
| 58 | STACNEG | 25.90 | 523.45 | 28.29 | 25.79 | 23.29 | 2.50 | 18.50 | 1309.04 | 50.76 |
| 59 | STACNEG | 18.40 | 727.85 | 20.16 | 17.49 | 17.32 | 2.67 | 36.10 | 1945.03 | 111.22 |
| 60 | STACNEG | 20.10 | 533.47 | 23.12 | 20.12 | 18.95 | 3.00 | 23.07 | 1602.92 | 79.69 |
| 61 | STACNEG | 31.70 | 370.55 | 28.05 | 25.37 | 24.03 | 2.69 | 13.21 | 995.04 | 39.23 |

| Sequence (Column 1) | Isolate (Column 2) | TTD (Column 3) | MaxART (Column 4) | Time MaxART (Column 5) | Time ART@50 (Column 6) | Time ART@20 (Column 7) | TimeMaxART - TimeART@50 (Column 8) | Ratio Mart_ TMAXA RT (Column 9) | Interval 50 (Column 10) | Int50_TT ART50 (Column 11) |
|---|---|---|---|---|---|---|---|---|---|---|
| 62 | STACNEG | 61.20 | 491.33 | 63.29 | 61.13 | 59.96 | 2.17 | 7.76 | 1065.06 | 17.42 |
| 63 | STACNEG | 17.80 | 516.47 | 20.05 | 17.88 | 16.55 | 2.17 | 25.76 | 1119.29 | 62.60 |
| 64 | STACNEG | 18.70 | 506.82 | 21.60 | 18.37 | 16.53 | 3.23 | 23.47 | 1637.18 | 89.13 |
| 65 | STACNEG | 39.80 | 365.48 | 43.92 | 39.69 | 39.02 | 4.23 | 8.32 | 1546.78 | 38.98 |
| 66 | STACNEG | 18.90 | 590.70 | 21.57 | 18.40 | 17.07 | 3.17 | 27.38 | 1871.04 | 101.66 |
| 67 | STACNEG | 19.10 | 460.67 | 21.29 | 18.62 | 17.62 | 2.67 | 21.64 | 1228.70 | 65.97 |
| 68 | STACNEG | 28.90 | 541.97 | 30.74 | 28.23 | 27.06 | 2.51 | 17.63 | 1360.02 | 48.17 |
| 69 | STACNEG | 22.90 | 472.97 | 26.14 | 22.97 | 20.63 | 3.17 | 18.10 | 1498.13 | 65.23 |
| 70 | STACNEG | 16.50 | 571.60 | 18.79 | 16.05 | 6.86 | 2.74 | 30.43 | 1564.30 | 97.46 |
| 71 | STACNEG | 23.90 | 454.45 | 26.98 | 23.48 | 22.48 | 3.50 | 16.84 | 1592.30 | 67.83 |
| 72 | STACNEG | 21.20 | 804.02 | 23.77 | 20.43 | 18.10 | 3.33 | 33.83 | 2681.00 | 131.20 |
| 73 | STACNEG | 80.30 | 265.65 | 90.39 | 83.72 | 79.37 | 6. 67 | 2.94 | 1772.18 | 21.17 |
| 74 | STACNEG | 30.90 | 606.87 | 34.49 | 30.15 | 27.65 | 4.34 | 17.60 | 2633.33 | 87.34 |
| 75 | STACNEG | 18.70 | 533.92 | 16.92 | 13.87 | 13.06 | 3.05 | 31.55 | 1626.53 | 117.24 |
| 76 | STACNEG | 23.70 | 575.67 | 25.78 | 22.93 | 21.26 | 2.85 | 22.33 | 1638.47 | 71.46 |
| 77 | STACNEG | 20.70 | 413.22 | 24.63 | 20.95 | 19.95 | 3.68 | 16.78 | 1522.14 | 72.67 |
| 78 | STACNEG | 23.40 | 816.38 | 25.48 | 22.61 | 19.93 | 2.87 | 32.04 | 2344.81 | 103.73 |
| 79 | STACNEG | 15.50 | 577.42 | 16.90 | 15.24 | 12.35 | 1. 67 | 34.16 | 962.67 | 63.18 |
| 80 | STACNEG | 15.20 | 900.37 | 18.29 | 14.89 | 13.70 | 3.40 | 49.24 | 3060.99 | 205.61 |
| 81 | STACNEG | 20.30 | 69.13 | 20.49 | 20.32 | 19.49 | 0.17 | 3.37 | 11.54 | 0.57 |
| 82 | STACNEG | 125.70 | 55.92 | 139.08 | 137.74 | 125.56 | 1.33 | 0.40 | 74.59 | 0.54 |
| 83 | STACNEG | 22.50 | 224.73 | 23.51 | 22.00 | 21.67 | 1.51 | 9.56 | 338.78 | 15.40 |
| 84 | STACNEG | 27.60 | 543.70 | 30.29 | 26.62 | 26.28 | 3.68 | 17.95 | 1998.21 | 75.07 |
| 85 | STACNEG | 14.70 | 709.97 | 17.09 | 14.42 | 13.42 | 2.67 | 41.54 | 1897.18 | 131.57 |
| 86 | STACNEG | 22.30 | 517.22 | 24.35 | 21.85 | NC | 2.50 | 21.24 | 1293.52 | 59.20 |
| 87 | PROTMIR | 13.70 | 885.83 | 15.73 | 12.89 | 7.04 | 2.84 | 56.33 | 2513.99 | 195.05 |
| 88 | KLEPNEP | 51.70 | 836.13 | 53.05 | 50.88 | 50.38 | 2.17 | 15.76 | 1811.81 | 35.61 |
| 89 | KLEPNEP | 10.80 | 1578.75 | 13.20 | 10.52 | 10.19 | 2.68 | 119.60 | 4224.10 | 401.38 |
| 90 | KLEPNEP | 13.80 | 1415.38 | 16.39 | 13.72 | 13.05 | 2.67 | 86.38 | 3775.53 | 275.22 |
| 91 | NEIMEN | 18.40 | 477.40 | 20.64 | 17.63 | NC | 3.01 | 23.13 | 1437.79 | 81.55 |

EP 2 913 404 B1

EP 2 913 404 B1

(continued)

| Sequence (Column 1) | Isolate (Column 2) | TTD (Column 3) | MaxART (Column 4) | Time MaxART (Column 5) | Time ART@50 (Column 6) | Time ART@20 (Column 7) | TimeMaxART - TimeART@50 (Column 8) | Ratio Mart_ TMAXA RT (Column 9) | Interval 50 (Column 10) | Int50_TT ART50 (Column 11) |
|---|---|---|---|---|---|---|---|---|---|---|
| 92 | ACINBAU | 11.60 | 226.03 | 13.21 | 11.37 | 10.70 | 1.84 | 17.11 | 416.96 | 36.68 |
| 93 | ACINBAU | 14.00 | 359.07 | 13.23 | 11.71 | 8.85 | 1.52 | 27.14 | 544.28 | 46.46 |

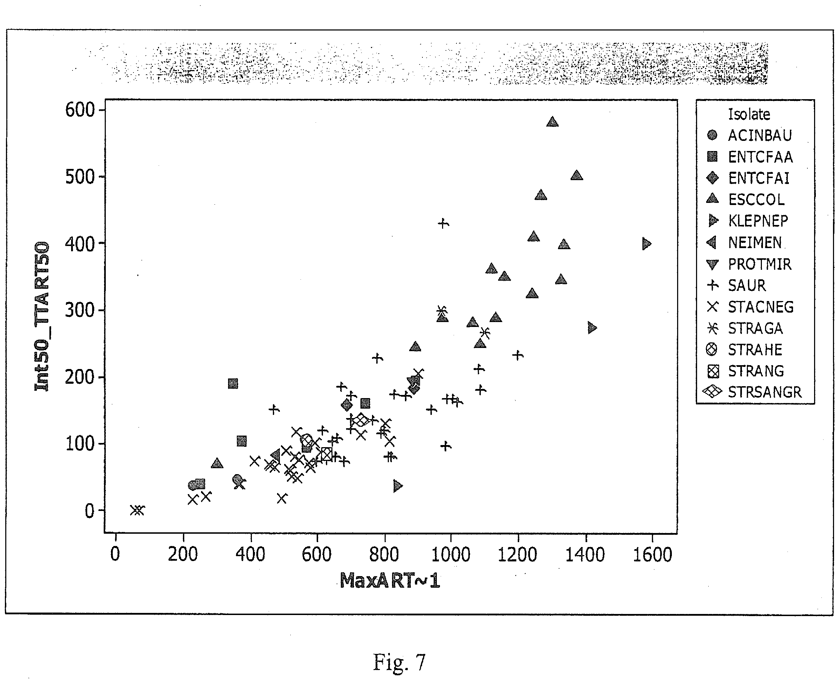

Figure 7 is a plot of the ratio of the extent of growth / versus maximum metabolic rate (defined as ARTmax Interval50 / TTART50 versus ARTmax for purposes of plotting Figure 7) for reference cultures containing microorganisms of known type. In Figure 7, the bacteria in the Enterobacteriaceae family (*e.g., E. coli* and *K. pneumoniae*) group together in the upper right hand corner of the figure. The Enterobacteriaceae were characterized as having a high ARTmax and a high Integral50 to TTART50 ratio (extent of growth as determined by Equation 3). There were some *streptococci* (STRAGA) that fit into this category. They can be distinguished by their relative NR change compared to the Enterobacteriaceae bacteria.

The next distinguishable group in Figure 7 was the *Staphylococcus aureus* that have an ARTmax value of greater than 600 and an Integral50 to TTART50 ratio greater than 80 (extent of growth as determined by Equation 3). This helped distinguish the *Staphylococcus aureus* from the coagulase negative *staphylococci* that typically have a lower ARTmax and Integral50 to TTART50 ratio.

The next distinguishable group in Figure 7 was the Acinetobacter (represented by *Acinetobacter baumannii* in this example). Microorganisms of this type had a low ARTmax value because of their obligately respiratory character and their preference for non carbohydrate substrates. Their NR curves were very characteristic of having an early rapid burst of growth followed by an early rapid cessation of growth the result is that they never achieve a sustained ARTmax.

## Claims

1. An apparatus for identifying a microorganism type in a culture in a vessel, the apparatus comprising a processor and a memory, coupled to the processor, the memory comprising:

    (A) electronically encoded instructions for obtaining a plurality of sets of values, each set of values in the plurality of sets of values comprising a maximum metabolic rate value and an extent of growth for a different microorganism type in a set of microorganism types; and
    (B) electronically encoded instructions for storing a lookup table that comprises matches between (i) the plurality of sets of values and (ii) a set of microorganism types, wherein, for each set of values in the plurality of sets of values there is a corresponding microorganism type in the set of microorganism types the memory of the apparatus further comprising a microorganism type determination module that comprises: (a) electronically encoded instructions for calculating a normalization relative value for each respective measurement in a plurality of measurements, taken at a different time points between a first time point and a second time point, between (i) the respective measurement and (ii) an initial biological state of the culture taken at an initial time point, thereby forming a plurality of normalization relative values wherein the biological state is a measure of the metabolic activity of the a culture as determined by, $CO_2$ concentration, $O_2$ concentration, pH, a rate of change in $CO_2$ concentration, a rate of change in $O_2$ concentration, or a rate of change in pH in the culture.; (b) electronically encoded instructions for determining, for each respective predetermined fixed interval of time points between the first time point and the second time point, a first derivative of the normalization relative values for measurements of the biological state in the respective predetermined fixed interval of time points, thereby forming a plurality of rate transformation values, wherein the plurality of rate transformation values comprises a plurality of sets of rate transformation values, wherein each respective set of rate transformation values in the plurality of sets of rate transformation values is for a different set of contiguous time points between the first time point and the second time point;
    (C) electronically encoded instructions for computing, for each respective set of rate transformation values in the plurality of sets of rate transformation values, an average relative transformation value as a measure of central tendency of each of the rate transformation values in the respective set of rate transformation values, thereby computing a plurality of average relative transformation values;
    (D) electronically encoded instructions for determining a maximum metabolic rate and an extent of growth from the plurality of normalization relative values and the plurality of average relative transformation values; and
    (E) electronically encoded instructions for comparing the maximum metabolic rate and the extent of growth with the lookup table that matches the maximum metabolic rate and the extent of growth to a microorganism type, thereby determining the microorganism type in the culture in the vessel.

2. A method of identifying a microorganism type in a culture in a vessel, the method comprising:

    (A) obtaining a plurality of measurements of the biological state of the culture in the vessel, wherein the biological state is a measure of the metabolic activity of the culture as determined by a rate of change in $CO_2$ concentration, a rate of change in $O_2$ concentration, or a rate of change in pH in the culture, each measurement in the plurality of measurements taken at a different time point between a first time point and a second time point;

(B) determining, for each respective predetermined fixed interval of time points between the first time point and the second time point, a first derivative of the measurements of the biological state in the respective predetermined fixed interval of time points, thereby forming a plurality of rate transformation values, wherein the plurality of rate transformation values comprises a plurality of sets of rate transformation values, wherein each respective set of rate transformation values in the plurality of sets of rate transformation values is for a different set of contiguous time points between the first time point and the second time point;

(C) computing, for each respective set of rate transformation values in the plurality of sets of rate transformation values, an average relative transformation value as a measure of central tendency of each of the rate transformation values in the respective set of rate transformation values, thereby computing a plurality of average relative transformation values;

(D) determining a maximum metabolic rate and an extent of growth from the plurality of normalization relative values and the plurality of average relative transformation values; and

(E) determining the microorganism type in the culture in the vessel from the maximum metabolic rate and the extent of growth.

3.  The method of claim 2, wherein the determining step (E) comprises comparing the maximum metabolic rate and the extent of growth with a lookup table that matches the maximum metabolic rate and the extent of growth to a microorganism type, thereby determining the microorganism type in the culture in the vessel.

**Patentansprüche**

1.  Vorrichtung zum Identifizieren eines Mikroorganismus-Typs in einer in einem Gefäß befindlichen Kultur, wobei die Vorrichtung einen Prozessor und einen Speicher aufweist, der mit dem Prozessor verbunden ist, wobei der Speicher aufweist:

(A) elektronisch kodierte Instruktionen zum Erhalt mehrerer Sätze von Werten, wobei jeder Satz von Werten in den mehreren Sätzen von Werten einen Maximalwert der metabolischen Rate und ein Maß an Wachstum für einen unterschiedlichen Mikroorganismus-Typ in einem Satz von Mikroorganismus-Typen aufweist; und

(B) elektronisch kodierte Instruktionen zum Speichern einer Lookup-Tabelle, die Entsprechungen zwischen (i) den mehreren Sätzen von Werten und (ii) einem Satz von Mikroorganismus-Typen aufweist, wobei für jeden Satz von Werten in den mehreren Sätzen von Werten ein entsprechender Mikroorganismus-Typ in dem Satz von Mikroorganismus-Typen existiert, wobei der Speicher der Vorrichtung ferner ein Mikroorganismus-Typ-Bestimmungsmodul enthält, das aufweist: (a) elektronisch kodierte Instruktionen zum Berechnen eines Normalisierungs-Relativwerts für jeden jeweiligen Messwert unter mehreren zu verschiedenen Zeitpunkten zwischen einem ersten Zeitpunkt und einem zweiten Zeitpunkt erfassten Messwerten, zwischen (i) dem jeweiligen Messwert und (ii) einem an einem Anfangszeitpunkt erfassten anfänglichen biologischen Zustand der Kultur, wodurch mehrere Normalisierungs-Relativwerte gebildet werden, wobei der biologische Zustand ein Maß der metabolischen Aktivität der Kultur ist, das bestimmt ist durch die $CO_2$-Konzentration, die $O_2$-Konzentration, den pH, eine Veränderungsrate in der $CO_2$-Konzentration, eine Veränderungsrate in der $O_2$-Konzentration, oder eine Veränderungsrate des pH in der Kultur; (b) elektronisch kodierte Instruktionen, um für jedes jeweilige vorbestimmte feste Intervall von Zeitpunkten zwischen dem ersten Zeitpunkt und dem zweiten Zeitpunkt einen ersten Ableitungswert der Normalisierungs-Relativwerte für die Messwerte des biologischen Zustands in dem jeweiligen vorbestimmten festen Intervall von Zeitpunkten zu bestimmen, wodurch mehrere Ratentransformationswerte gebildet werden, wobei die mehreren Ratentransformationswerte mehrere Sätze von Ratentransformationswerten aufweisen, wobei jeder jeweilige Satz von Ratentransformationswerten in den mehreren Sätzen von Ratentransformationswerten für einen unterschiedlichen Satz von benachbarten Zeitpunkten zwischen dem ersten Zeitpunkt und dem zweiten Zeitpunkt vorgesehen ist;

(C) elektronisch kodierte Instruktionen, um für jeden jeweiligen Satz von Ratentransformationswerten in den mehreren Sätzen von Ratentransformationswerten einen mittleren Relativ-Transformationswert als Maß einer zentralen Tendenz jedes der Ratentransformationswerte in dem jeweiligen Satz von Ratentransformationswerten zu berechnen, wodurch mehrere mittlere Relativ-Transformationswerte berechnet werden;

(D) elektronisch kodierte Instruktionen zum Bestimmen einer maximalen metabolischen Rate und eines Maßes an Wachstum aus den mehreren Normalisierungs-Relativwerten und den mehreren mittleren Relativ-Transformationswerten; und

(E) elektronisch kodierte Instruktionen zum Vergleichen der maximalen metabolischen Rate und des Maßes an Wachstum mit Hilfe der Lookup-Tabelle, welche die maximale metabolische Rate und das Maß an Wachstum mit einem Mikroorganismus-Typ in Beziehung setzt, wodurch der Mikroorganismus-Typ in der in dem Gefäß befindlichen Kultur bestimmt wird.

2. Verfahren zum Identifizieren eines Mikroorganismus-Typs in einer in einem Gefäß befindlichen Kultur, wobei das Verfahren umfasst:

(A) Erhalt mehrerer Messwerte des biologischen Zustands der in dem Gefäß befindlichen Kultur, wobei der biologische Zustand ein Maß der metabolischen Aktivität der Kultur ist, das bestimmt ist durch eine Veränderungsrate der $CO_2$-Konzentration, eine Veränderungsrate der $O_2$-Konzentration, oder eine Veränderungsrate des pH in der Kultur, wobei jeder Messwert unter den mehreren Messwerten an einem unterschiedlichen Zeitpunkt zwischen einem ersten Zeitpunkt und einem zweiten Zeitpunkt erfasst wird;
(B) für jedes jeweilige vorbestimmte feste Intervall von Zeitpunkten zwischen dem ersten Zeitpunkt und dem zweiten Zeitpunkt, Bestimmen eines ersten Ableitungswerts der Messwerte des biologischen Zustands in dem jeweiligen vorbestimmten festen Intervall von Zeitpunkten, wodurch mehrere Ratentransformationswerte gebildet werden, wobei die mehreren Ratentransformationswerte mehrere Sätze von Ratentransformationswerten aufweisen, wobei jeder jeweilige Satz von Ratentransformationswerten in den mehreren Sätzen von Ratentransformationswerten für einen unterschiedlichen Satz von benachbarten Zeitpunkten zwischen dem ersten Zeitpunkt und dem zweiten Zeitpunkt vorgesehen ist;
(C) für jeden jeweiligen Satz von Ratentransformationswerten in den mehreren Sätzen von Ratentransformationswerten, Berechnen eines mittleren Relativ-Transformationswerts als Maß einer zentralen Tendenz jedes der Ratentransformationswerte in dem jeweiligen Satz von Ratentransformationswerten, wodurch mehrere mittlere Relativ-Transformationswerte berechnet werden;
(D) Bestimmen einer maximalen metabolischen Rate und eines Maßes an Wachstum aus den mehreren Normalisierungs-Relativwerten und den mehreren mittleren Relativ-Transformationswerten; und
(E) Bestimmen des Mikroorganismus-Typs in der in dem Gefäß befindlichen Kultur aus der maximalen metabolischen Rate und dem Maß an Wachstum.

3. Verfahren nach Anspruch 2, bei dem der Bestimmungs-Schritt (E) das Vergleichen der maximalen metabolischen Rate und des Maßes an Wachstum mit Hilfe einer Lookup-Tabelle umfasst, welche die maximale metabolische Rate und das Maß an Wachstum mit einem Mikroorganismus-Typ in Beziehung setzt, wodurch der Mikroorganismus-Typ in der in dem Gefäß befindlichen Kultur bestimmt wird.

**Revendications**

1. Appareil pour identifier un type de microorganisme dans une culture dans un récipient, l'appareil comprenant un processeur et une mémoire, couplée au processeur, la mémoire comprenant :

(A) des instructions codées électroniquement pour obtenir une pluralité d'ensembles de valeurs, chaque ensemble de valeurs dans la pluralité d'ensembles de valeurs comprenant une valeur de vitesse de métabolisme maximale et un degré de croissance pour un type de microorganisme différent dans un ensemble de types de microorganismes ; et
(B) des instructions codées électroniquement pour stocker une table de consultation qui comprend des correspondances entre (i) la pluralité d'ensembles de valeurs et (ii) un ensemble de types de microorganismes, où, pour chaque ensemble de valeurs dans la pluralité d'ensembles de valeurs, il existe un type de microorganisme correspondant dans l'ensemble de types de microorganismes, la mémoire de l'appareil comprenant en outre un module de détermination de type de microorganisme qui comprend : (a) des instructions codées électroniquement pour calculer une valeur relative de normalisation pour chaque mesure respective dans une pluralité de mesures, prise à un instant différent entre un premier instant et un deuxième instant, entre (i) la mesure respective et (ii) un état biologique initial de la culture pris à un instant initial, formant ainsi une pluralité de valeurs relatives de normalisation où l'état biologique est une mesure de l'activité métabolique de la culture telle que déterminée par, la concentration en $CO_2$, la concentration en $O_2$, le pH, un taux de changement de concentration en $CO_2$, un taux de changement de concentration en $O_2$, ou un taux de changement de pH dans la culture ; (b) des instructions codées électroniquement pour déterminer, pour chaque intervalle fixe prédéterminé respectif d'instants entre le premier instant et le deuxième instant, une première dérivée des valeurs relatives de normalisation pour des mesures de l'état biologique dans l'intervalle fixe prédéterminé respectif d'instants, formant ainsi une pluralité de valeurs de transformation de taux, où la pluralité de valeurs de transformation de taux comprend une pluralité d'ensembles de valeurs de transformation de taux, où chaque ensemble respectif de valeurs de transformation de taux dans la pluralité d'ensembles de valeurs de transformation de taux est pour un ensemble différent d'instants contigus entre le premier instant et le deuxième instant ;

(C) des instructions codées électroniquement pour calculer, pour chaque ensemble respectif de valeurs de transformation de taux dans la pluralité d'ensembles de valeurs de transformation de taux, une valeur de transformation relative moyenne comme une mesure de tendance centrale de chacune des valeurs de transformation de taux dans l'ensemble respectif de valeurs de transformation de taux, calculant ainsi une pluralité de valeurs de transformation relatives moyennes ;

(D) des instructions codées électroniquement pour déterminer une vitesse de métabolise maximale et un degré de croissance à partir de la pluralité de valeurs relatives de normalisation et de la pluralité de valeurs de transformation relatives moyennes ; et

(E) des instructions codées électroniquement pour comparer la vitesse de métabolisme maximale et le degré de croissance à la table de consultation qui fait correspondre la vitesse de métabolisme maximale et le degré de croissance à un type de microorganisme, déterminant ainsi le type de microorganisme dans la culture dans le récipient.

2. Procédé d'identification d'un type de microorganisme dans une culture dans un récipient, le procédé comprenant le fait :

(A) d'obtenir une pluralité de mesures de l'état biologique de la culture dans le récipient, où l'état biologique est une mesure de l'activité métabolique de la culture telle que déterminée par un taux de changement de concentration en $CO_2$, un taux de changement de concentration en $O_2$, ou taux de changement de pH dans la culture, chaque mesure dans la pluralité de mesures étant prise à un instant différent entre un premier instant et un deuxième instant ;

(B) de déterminer, pour chaque intervalle fixe prédéterminé respectif d'instants entre le premier instant et le deuxième instant, une première dérivée des mesures de l'état biologique dans l'intervalle fixe prédéterminé respectif d'instants, formant ainsi une pluralité de valeurs de transformation de taux, où la pluralité de valeurs de transformation de taux comprend une pluralité d'ensembles de valeurs de transformation de taux, où chaque ensemble respectif de valeurs de transformation de taux dans la pluralité d'ensembles de valeurs de transformation de taux est pour un ensemble différent d'instants contigus entre le premier instant et le deuxième instant ;

(C) de calculer, pour chaque ensemble respectif de valeurs de transformation de taux dans la pluralité d'ensembles de valeurs de transformation de taux, une valeur de transformation relative moyenne comme une mesure de tendance centrale de chacune des valeurs de transformation de taux dans l'ensemble respectif de valeurs de transformation de taux, calculant ainsi une pluralité de valeurs de transformation relatives moyennes ;

(D) de déterminer une vitesse de métabolisme maximale et un degré de croissance à partir de la pluralité de valeurs relatives de normalisation et de la pluralité de valeurs de transformation relatives moyennes ; et

(E) de déterminer le type de microorganisme dans la culture dans le récipient à partir de la vitesse de métabolisme maximale et du degré de croissance.

3. Procédé de la revendication 2, dans lequel l'étape de détermination (E) comprend le fait de comparer la vitesse de métabolisme maximale et le degré de croissance à une table de consultation qui fait correspondre la vitesse de métabolisme maximale et le degré de croissance à un type de microorganisme, déterminant ainsi le type de microorganisme dans la culture dans le récipient.

11

Power
Source

CPU

Communications
Circuitry

34 (e.g. 202)

Sensor

24

22

20

30

32

26

28

12

Controller

14

36

| Operating System | | | 40 |
| File system | | | 42 |
| Culture type determination module | | | 44 |
| Biological state data structure | | | 46 |
| | Initial biological state of the culture | | 48 |
| | Biological state of the culture | | 50-1 |
| | • • • | | |
| | Biological state of the culture | | 50-N |
| Lookup table | | | 54 |

| Set of values 56-1 (maximum metabolic rate value 57-1 / extent of growth 58-1) | Microorganism type 59-1 |
|---|---|
| • • • | |
| Set of values 56-N (maximum metabolic rate value 57-N / extent of growth 58-N) | Microorganism type 59-N |

| Set of rate transformation values 1 | 60-1 |
| Rate transformation value 1-1 | 62-1-1 |
| Rate transformation value 1-2 | 62-1-2 |
| • • | |
| Rate transformation value 1-N | 62-1-N |
| • • | |
| Set of rate transformation values M | 60-M |
| Rate transformation value M-1 | 62-M-1 |
| Rate transformation value M-2 | 62-M-2 |
| • • | |
| Rate transformation value M-N | 62-M-N |
| Average relative transformation value 1 | 66-1 |
| • • | |
| Average relative transformation value M | 66-M |
| Identification of microorganism type in culture | 68 |
| • • • | |

Fig. 1

Fig. 2

Measure initial biological state of culture with unidentified microorganisms upon initialization ─302

Standardize and store the measured initial biological state of the culture upon initialization (*e.g.* to one hundred percent or some other predetermined value) ─304

Advance time step *t* ─306    ◄── ( B )

Measure and store the normalization relative (NR) value of the culture, the spectroscopic state of the culture after initialization ─308

Has first predetermined fixed interval elapsed? ─310    No

Yes

Calculate and store the rate transformation value (RT), which is the first derivative of the NR values over the past predetermined fixed time interval (*e.g.* the change in NR over the previous 70 minutes of measurement) ─312

Have at least a predetermined number of RT values been measured? ─314    No

Yes

Calculate and store the average relative transformation (average rate change) (ART) value (first derivative), as the average of the previous predetermined number of RT values that have been measured and stored ─316

Have at least a predetermined number of ART values been measured? ─318    No

Yes

( A )

Fig. 3A

A

Determine the maximum metabolic rate value 57 ———320

Determine the extent of growth of the culture 58 ———322

Compare the maximum metabolic rate value 57 and the extent of growth of the culture 58 with a lookup table that matches the maximum metabolic rate 57 and the extent of growth of the culture 58 to a microorganism type, thereby determining the microorganism type in the culture in the vessel. ———324

Fig. 3B

Fig. 4

Fig. 5

EP 2 913 404 B1

Fig. 6

EP 2 913 404 B1

Fig. 7

EP 2 913 404 B1

## REFERENCES CITED IN THE DESCRIPTION

### Patent documents cited in the description

- US 20020155424 A, Pitner
- EP 0697460 A
- US 6432697 B
- US 6617127 B
- US 6096272 A
- US 4889992 A
- WO 2006071800 A
- US 6900030 B

### Non-patent literature cited in the description

- **STANIER et al.** The Microbial World. Prentice-Hall, 1986, 10-20, 33-37, 190-195
- **STANIER et al.** The Microbial World. Prentice-Hall, 1986, 10-20, 33-37, 190-195
- **STANIER et al.** The Microbial Would. Prentice-Hall, 1986
- **OBEROI et al.** Comparison of rapid colorimetric method with conventional method in the isolation of mycobacterium tuberculosis. *Indian J Med Microbiol,* 2004, vol. 22, 44-46